# EUROPÄISCHE PATENTSCHRIFT

(11) **EP 2 653 120 B1**
(45) Veröffentlichungstag und Bekanntmachung des Hinweises auf die Patenterteilung: **27.05.2020**
(21) Anmeldenummer: 13001867.4
(22) Anmeldetag: 11.04.2013
(51) Int. Cl.: A61B 17/29, A61B 17/3201, A61B 17/00, A61B 17/295, A61B 18/14

(54) **Handhabungseinrichtung für ein medizinisches Instrument**
Handle for a medical instrument
Dispositif de manipulation pour un instrument médical

(30) Priorität: 18.04.2012 DE 102012007650
(43) Veröffentlichungstag der Anmeldung: 23.10.2013
(73) Patentinhaber: Karl Storz SE & Co. KG, 78532 Tuttlingen (DE)
(72) Erfinder: Merz, Robin, 78532 Tuttlingen (DE); Schneider, Sven, 78532 Tuttlingen (DE); Jochen, Stefan, 78532 Tuttlingen (DE)

(56) Entgegenhaltungen:
- DE-A1- 19 852 682
- DE-T2-602005 001 056
- US-A- 5 913 874
- US-B1- 6 358 267

## Beschreibung

Die vorliegende Erfindung ist auf eine Handhabungseinrichtung für ein medizinisches Instrument und auf ein medizinisches Instrument bezogen, insbesondere auf die lösbare mechanische Kopplung der Handhabungseinrichtung mit Übertragungseinrichtungen in einem Schaft.

Die Erwartungen an medizinische Instrumente, insbesondere an medizinische Instrumente für mikroinvasive Eingriffe steigen beständig. Bereits in Vielfalt angeboten und weit verbreitet sind medizinische Instrumente mit einem Werkzeug mit Greif- oder Schneidefunktion am distalen Ende. Zunehmend kommen weitere Funktionen und Freiheitgrade hinzu, beispielsweise eine Rotation des Werkzeugs um die Längsachse des Schafts, eine Abwinkelbarkeit des Schafts proximal des Werkzeugs oder eine zweite, unabhängig steuerbare Wirkeinrichtung am Werkzeug. Zur Steuerung dieser weiteren Funktionen oder Freiheitsgrade kann ein zweites Übertragungselement im Schaft des medizinischen Instruments vorgesehen sein, beispielsweise eine zweite Übertragungsstange.

Bei wiederverwendbaren medizinischen Instrumenten ist für die Reinigung eine möglichst weitgehende Zerlegbarkeit erforderlich. Insbesondere ist bei vielen medizinischen Instrumenten die Handhabungseinrichtung vom proximalen Ende des Schafts trennbar. Dabei kann beispielsweise eine Rastverbindung zwischen dem proximalen Ende des Schafts bzw. des Außenschafts einerseits und der Handhabungseinrichtung andererseits durch manuellen Druck auf einen Entriegelungsknopf oder Betätigung einer anderen Betätigungseinrichtung entriegelt werden. Bei von der Anmelderin hergestellten und unter der Bezeichnung "Clickline" vertriebenen medizinischen Instrumenten gelangen das proximale Ende der Übertragungsstange und ein mit dieser gekoppelter Hebel der Halteeinrichtung beim Herausziehen des Schafts nach distal aus der Handhabungseinrichtung in Positionen, in denen sie nicht mehr miteinander gekoppelt sind. Die Kopplung zwischen dem proximalen Ende der Übertragungsstange und der Handhabungseinrichtung bzw. einem Hebel an der Handhabungseinrichtung ist somit lösbar, wenn die Kopplung zwischen dem proximalen Ende des Schafts und der Handhabungseinrichtung gelöst ist.

Die von "Clickline"-Produkten bekannte Lösbarkeit bzw. Trennbarkeit der proximalen Enden des Außenschafts und der Übertragungsstange einerseits von der Handhabungseinrichtung andererseits kann jedoch auf manch andere medizinische Instrumente nicht oder nicht ohne Weiteres übertragen werden, insbesondere wenn im Außenschaft mehr als eine unabhängig bewegbare Übertragungseinrichtung angeordnet ist. Eine Alternative besteht darin, für jede Kopplung eine separate Betätigungseinrichtung vorzusehen. Im Fall eines medizinischen Instruments mit einem Außenschaft und zwei Übertragungsstangen oder anderen Übertragungseinrichtungen sind also drei Betätigungseinrichtungen vorzusehen, mittels derer manueller Betätigung jeweils eine Kopplung zwischen dem proximalen Ende des Außenschafts bzw. einer Übertragungsstange einerseits und der Handhabungseinrichtung andererseits lösbar ist.

In DE 198 52 682 A1 ist ein chirurgisches Schienenschaftinstrument beschrieben. Ein Branchenelement 16 umfasst eine stationäre Branche 18, einen Arm 20 und eine bewegbare Branche 22, die mittels eines lösbaren Drehzapfens 52 an dem Arm 20 befestigt ist. Ein Führungselement 12 ist über einen Schwenkzapfen 54 mit dem Arm 20 verbunden und relativ zu diesem schwenkbar. Ein Rasthaken 42 an einem federbelasteten Verschlusselement 26 kann mit einem Verriegelungshaken an dem Führungselement 12 in Eingriff gebracht werden, um das Führungselement mit der stationären Branche 18 und dem Arm 20 zu verbinden.

Das relativ zu der stationären Branche 18 und dem Arm 20 um den Schwenkzapfen 54 schwenkbare Führungselement 12 kann einen Arbeitsaufsatz 14 aufnehmen, der von proximal in das Führungselement 12 eingeführt werden kann, wenn das Führungselement 12 von der stationären Branche 18 und dem Arm 20 weg geschwenkt ist. Der Arbeitsaufsatz 14 umfasst an seinem proximalen Ende einen Anschlag 32 und ein Lochrad 34. Wenn das Führungselement 12 mit der stationären Branche 18 und dem Arm 20 verbunden ist, liegt ein Schiebeteil 24 der bewegbaren Branche 22 an dem Anschlag 32 an. Wenn das Führungselement 12 mit der stationären Branche 18 und dem Arm 20 verbunden ist, kann ferner ein Zapfen 36 an einem federbelasteten Auslösehebel 38 in das Lochrad 34 eingreifen. Wenn der Zapfen 36 nicht in das Lochrad 34 eingreift, kann die Winkelposition des Arbeitsaufsatzes 14 verändert werden.

In DE 60 2005 001 056 T2 ist ein chirurgisches Mehrzweckinstrument 40 beschrieben. Eine Handgriff-Baueinheit 46 kann verschiedene chirurgische Steckmodule 50, 52 aufnehmen. Dazu weist die Handgriff-Baueinheit 46 eine Dockingstation 86 mit einem Gehäuse 96 und einem Deckel 102 auf. Beim Einlegen eines chirurgischen Steckmoduls 50, 52 in die Dockingstation 86 entstehen mehrere mechanische und elektrische Verbindungen zwischen der Handgriff-Baueinheit 46 und dem chirurgischen Steckmodul 50, 52.

Eine Aufgabe der vorliegenden Erfindung besteht darin, eine verbesserte Handhabungseinrichtung für ein medizinisches Instrument und ein verbessertes medizinisches Instrument zu schaffen.

Diese Aufgabe wird durch die Gegenstände der unabhängigen Ansprüche gelöst.

Weiterbildungen sind in den abhängigen Ansprüchen angegeben.

Ausführungsbeispiele der vorliegenden Erfindung beruhen auf der Idee, an einer Handhabungseinrichtung mehrere Kopplungseinrichtungen zur lösbaren mechanischen Kopplung je einer zugeordneten Betätigungseinrichtung mit einer zugeordneten Übertragungseinrichtung in einem mit der Handhabungseinrichtung zu verbindenden Außenschaft so auszubilden, dass jede Kopplungseinrichtung eine zugeordnete Entkopplungsposition aufweist, in der die zugeordnete Betätigungseinrichtung von der zugeordneten Übertragungseinrichtung entkoppelt ist, wobei die Entkopplungspositionen der Kopplungseinrichtungen nur dann erreichbar sind, wenn der Außenschaft nicht in der vorgesehenen Weise mit der Handhabungseinrichtung verbunden ist.

Bei Betrachtung der eingangs erwähnten "Clickline"-Produkte entstand der Eindruck, dass die dort zu beobachtende automatische Kopplung und Entkopplung des proximalen Endes der Übertragungsstange mit einer hebelförmigen Betätigungseinrichtung nicht auf medizinische Instrumente mit zwei oder mehr Übertragungseinrichtungen übertragbar ist. Insbesondere stellt die lösbare mechanische Kopplung der zum Schwenken von einem oder zwei Maulteilen vorgesehenen Übertragungsstange mit einer hebelförmigen Betätigungseinrichtung bei den "Clickline"-Produkten insofern einen Spezialfall dar, als diese hebelförmige Betätigungseinrichtung ohne Weiteres durch eine Feder in die Position vorgespannt bzw. bewegt werden kann, in der eine Kopplung und Entkopplung von Übertragungsstange und Betätigungseinrichtung möglich ist. Dies liegt daran, dass diese Position einer offenen Position bzw. Stellung von Maulteilen am Werkzeug am distalen Ende des Schafts entspricht. Bei anderen Funktionen und Freiheitsgraden ist eine Bevorzugung einer bestimmten Position bzw. Stellung durch Federkraft in der Regel unerwünscht.

Die hier beschriebenen Varianten und Ausführungsbeispiele belegen, dass tatsächlich eine triviale Übertragung der von den "Clickline"-Produkten bekannten Kopplung der Übertragungsstange mit der zugeordneten Betätigungseinrichtung auf die Kopplung weiterer Übertragungseinrichtungen mit zugeordneten Betätigungseinrichtungen nicht ohne möglich ist, sondern zusätzliche konstruktive Merkmale erforderlich sind. Dann ist jedoch erfreulicherweise eine ähnlich einfache Handhabung beim Zusammensetzen und Zerlegen eines medizinischen Instruments realisierbar.

Eine Handhabungseinrichtung für ein medizinisches Instrument umfasst einen Grundkörper mit einer Kupplung zur lösbaren mechanischen Verbindung mit einem proximalen Ende eines Außenschafts, eine erste Betätigungseinrichtung, die relativ zum Grundkörper bewegbar ist, eine zweite Betätigungseinrichtung, die relativ zum Grundkörper bewegbar ist, eine erste Kopplungseinrichtung zur Kopplung der ersten Betätigungseinrichtung mit einer ersten Übertragungseinrichtung zum Übertragen zumindest entweder einer Kraft oder eines Drehmoments und eine zweite Kopplungseinrichtung zur Kopplung der zweiten Betätigungseinrichtung mit einer zweiten Übertragungseinrichtung zum Übertragen zumindest entweder einer Kraft oder eines Drehmoments, wobei die erste Kopplungseinrichtung und die zweite Kopplungseinrichtung jeweils eine Entkopplungsposition aufweisen, in der die zugeordnete Betätigungseinrichtung von der zugeordneten Übertragungseinrichtung entkoppelt ist, und wobei die Entkopplungspositionen beider Kopplungseinrichtungen nur dann erreichbar sind, wenn der Außenschaft nicht in der für die Verwendung des medizinischen Instruments vorgesehenen Weise mit der Kupplung am Grundkörper verbunden ist.

Die Handhabungseinrichtung ist insbesondere für ein mikroinvasiv-chirurgisches Instrument bzw. zur Bildung eines mikroinvasiv-chirurgischen Instruments aus der Handhabungseinrichtung, einem Werkzeug und einem Außenschaft, der die erste Betätigungseinrichtung und die zweite Betätigungseinrichtung aufnimmt, vorgesehen und ausgebildet. Dabei sind insbesondere die erste Übertragungseinrichtung eine Übertragungsstange und die zweite Übertragungseinrichtung ein Innenschaft, wobei der Innenschaft und die Übertragungsstange koaxial im Außenschaft angeordnet sind. Außenschaft und Übertragungseinrichtungen können jeweils gerade oder gekrümmt, starr oder biegeflexibel sein. Die Übertragungseinrichtungen sind insbesondere jeweils zum Übertragen zumindest entweder einer Kraft oder eines Drehmoments zu einem zugeordneten Werkzeug am distalen Ende des Schafts, zum Schwenken eines Maulteils oder zum Bewegen eines Messers oder Skalpells ausgebildet.

Der Grundkörper der Handhabungseinrichtung kann ein- oder mehrteilig ausgebildet sein. Die erste Betätigungseinrichtung und die zweite Betätigungseinrichtung sind jeweils insbesondere relativ zum Grundkörper schwenkbar oder translatorisch bewegbar. Die erste Betätigungseinrichtung ist beispielsweise zum Steuern des Öffnens und Schließens von greifenden oder schneidenden Maulteilen ausgebildet. Die zweite Betätigungseinrichtung ist beispielsweise zur Steuerung der Bewegung eines Skalpells ausgebildet.

In der bei medizinischen Eingriffen verwendbaren Konfiguration des medizinischen Instruments ist der Außenschaft in einer vorgesehenen Weise mit dem Grundkörper verbunden, insbesondere in vorgesehener Position relativ zum Grundkörper angeordnet und in dieser Position durch die Kupplung am Grundkörper gehalten. Die Kupplung umfasst insbesondere eine Ausnehmung, deren geometrische Gestalt dem proximalen Ende des Außenschafts entspricht. Die Kupplung kann in der Art einer Bajonettkupplung ausgebildet sein, ein Gewinde und/oder einen Riegel zum Verriegelung des Außenschafts in der vorgesehenen Position relativ zum Grundkörper der Handhabungseinrichtung umfassen.

Insbesondere können die erste Kopplungseinrichtung ihre zugeordnete Entkopplungsposition und die zweite Kopplungseinrichtung ihre zugeordnete Entkopplungsposition dann einnehmen, wenn der Außenschaft vom Grundkörper der Handhabungseinrichtung getrennt bzw. von der Position hinreichend beabstandet ist, in der er mit der Kupplung am Grundkörper lösbar, aber starr mechanisch verbunden werden kann.

Bei einer Handhabungseinrichtung, wie sie hier beschrieben ist, ist insbesondere die zweite Kopplungseinrichtung in jeder Position zumindest entweder von dem proximalen Ende eines mit der Handhabungseinrichtung verbundenen Außenschafts oder von dem proximalen Ende einer mit der ersten Betätigungseinrichtung gekoppelten ersten Übertragungseinrichtung elektrisch isoliert.

Die elektrische Isolation der zweiten Kopplungseinrichtung von dem eine elektrisch leitfähige Oberfläche aufweisenden proximalen Ende des Außenschafts und/oder von dem eine elektrisch leitfähige Oberfläche aufweisenden proximalen Ende der ersten Übertragungseinrichtung schließt insbesondere ein, dass für die in der Elektrochirurgie bzw. zur Elektrokauterisation üblichen elektrischen Spannungen ausreichende Kriechstrecken vorliegen.

Dass die zweite Kopplungseinrichtung in jeder Position vom proximalen Ende des Außenschafts und/oder vom proximalen Ende der ersten Übertragungseinrichtung elektrisch isoliert ist, ermöglicht eine Verwendung der Handhabungseinrichtung für ein bipolares elektrochirurgisches Instrument, bei dem der Außenschaft und die erste Übertragungseinrichtung mit zwei unterschiedlichen Polen verbunden bzw. zur Übertragung der beiden unterschiedlichen elektrischen Potentiale vorgesehen sind. Eine Übertragungsstange oder ein Innenschaft kann oft ohne Weiteres mittels eines Strumpfschlauchs nach außen isoliert werden. Am proximalen Ende weist eine Übertragungsstange oder ein Innenschaft jedoch oft blanke metallische Oberflächen auf, die aufgrund ihrer Härte und ihrer Verschleißeigenschaften zur mechanischen Kopplung mit der zugeordneten Kopplungseinrichtung der Handhabungseinrichtung besonders geeignet sind. Entsprechendes gilt für die Kupplung am Grundkörper und für die Kopplungseinrichtungen der Handhabungseinrichtung: sie weisen in der Regel aufgrund der erwünschten Härte und aufgrund der erwünschten Verschleißeigenschaften metallische und damit elektrisch leitfähige Oberflächen auf. Die jederzeitige elektrische Isolation der zweiten Kopplungseinrichtung vom proximalen Ende des Außenschafts und/oder vom proximalen Ende der ersten Übertragungseinrichtung schließt insbesondere eine entsprechende elektrische Isolation der zweiten Kopplungseinrichtung von der Kupplung am Grundkörper und/oder von der ersten Kopplungseinrichtung bzw. von deren elektrisch leitfähigen Bestandteilen in jeder Position der zweiten Kopplungseinrichtung ein.

Bei einer Handhabungseinrichtung, wie sie hier beschrieben ist, sind insbesondere die erste Betätigungseinrichtung zum Öffnen und Schließen von Maulteilen eines ersten Werkzeugs und die zweite Betätigungseinrichtung zum Bewegen eines zweiten Werkzeugs am distalen Ende eines mit der Handhabungseinrichtung zu koppelnden Schafts ausgebildet.

Das erste Werkzeug ist insbesondere eine Zange, eine Schere oder ein anderes Greif- oder Schneidewerkzeug mit zwei oder mehr Maulteilen, von denen mindestens eines relativ zu dem oder den anderen Maulteilen schwenkbar ist. Das erste Werkzeug ist insbesondere ferner zur monopolaren oder bipolaren Elektrochirurgie ausgebildet. Das zweite Werkzeug ist insbesondere ein Messer bzw. Skalpell, das in einem Kanal zwischen den geschlossenen Maulteilen des als Zange ausgebildeten ersten Werkzeugs in Längsrichtung verschiebbar ist. Damit kann beispielsweise ein Gefäßes nach dem Greifen, Abquetschen und elektrochirurgischen Verschließen durchtrennt werden.

Bei einer Handhabungseinrichtung, wie sie hier beschrieben ist, sind insbesondere die erste Betätigungseinrichtung proximal eines feststehenden Griffteils der Handhabungseinrichtung und die zweite Betätigungseinrichtung distal des feststehenden Griffteils angeordnet.

Insbesondere sind das feststehende Griffteil in einem Winkel zwischen 50° und 90° zur Längsachse eines mit der Handhabungseinrichtung zu verbindenden Außenschafts angeordnet, die erste Betätigungseinrichtung um eine Achse senkrecht zu der Längsachse schwenkbar und das feststehende Griffteil und die erste Betätigungseinrichtung ähnlich wie die Griffe einer Schere ausgebildet und mittels einer Hand greif- und bewegbar. Die zweite Betätigungseinrichtung umfasst insbesondere einen Schieber, einen Hebel, einen Zughebel oder eine Drucktaste. Die zweite Betätigungseinrichtung ist insbesondere so angeordnet, dass sie ohne Weiteres mit dem Zeigefinger einer Hand, deren übrige Finger das feststehende Griffteil und die erste Betätigungseinrichtung in der vorgesehenen Weise greifen, betätigt werden kann. Eine Handhabungseinrichtung, bei der die erste Betätigungseinrichtung proximale und die zweite Betätigungseinrichtung distal des feststehenden Griffteils angeordnet ist, kann somit eine hinsichtlich der Ergonomie und hinsichtlich der medizinischem Personal vertrauten Handhabung vorteilhafte Ähnlichkeit mit herkömmlichen Handhabungseinrichtungen, insbesondere denen der oben genannten "Clickline"-Produkte, aufweisen.

Bei einer Handhabungseinrichtung, wie sie hier beschrieben ist, umfasst die zweite Kopplungseinrichtung insbesondere einen Schlitten, der mittels der zweiten Betätigungseinrichtung entlang eines vorbestimmten Pfads verschiebbar ist.

Ein Schlitten ist insbesondere ein Bauteil, das durch Gleit- und/oder Wälzlager an einer oder mehreren Schienen oder anderen Führungseinrichtungen geführt ist, wobei die Führungseinrichtungen den vorbestimmten Pfad definieren. Von Spiel und elastischer Verformung abgesehen, weist der Schlitten lediglich einen Freiheitsgrad auf, nämlich die Bewegung entlang des vorbestimmten Pfads. Der vorbestimmte Pfad ist insbesondere gerade und insbesondere parallel zur Längsachse vom Außenschaft und Übertragungseinrichtungen, im Falle eines gekrümmten Außenschafts: parallel zur Längsachse des Außenschafts an dessen proximalem Ende. Die Entkopplungsposition der zweiten Kopplungseinrichtung ist insbesondere am distalen Ende des vorbestimmten Pfads des Schlittens, wobei die zweite Kopplungseinrichtung so ausgebildet sein kann, dass die zweite Übertragungseinrichtung und die zweite Kopplungseinrichtung in jeder Position der Kopplungseinrichtung gekoppelt werden können.

Der Schlitten kann eine konstruktiv und fertigungstechnisch einfach realisierbare Option darstellen. Beispielsweise greifen gegenüberliegende Ränder des Schlittens oder Stege oder Zapfen oder Stifte oder andere konvexe Bereiche an gegenüberliegenden Seiten des Schlittens in korrespondierende Nuten am Grundkörper der Handhabungseinrichtung ein. Alternativ können umgekehrt Stege am Grundkörper der Handhabungseinrichtung in Nuten an gegenüberliegenden Seiten des Schlittens eingreifen.

Bei einer Handhabungseinrichtung, wie sie hier beschrieben ist, umfasst die zweite Kopplungseinrichtung insbesondere einen Riegel zum formschlüssigen Halten des proximalen Endes der zweiten Übertragungseinrichtung an dem Schlitten, wobei der Grundkörper ausgebildet ist, um bei Annäherung der zweiten Kopplungseinrichtung an die Entkopplungsposition den Riegel in eine Entriegelungsposition zu bewegen, in der der Riegel das proximale Ende der zweiten Übertragungseinrichtung nicht mehr formschlüssig hält.

Der Riegel ist insbesondere an oder in dem Schlitten angeordnet und an dem Schlitten so geführt, dass er relativ zum Schlitten entlang eines vorbestimmten Pfads bewegbar ist.

Bei einer Handhabungseinrichtung, wie sie hier beschrieben ist, sind insbesondere der Schlitten in einer ersten Richtung bewegbar und der Riegel relativ zu dem Schlitten in einer zweiten Richtung senkrecht zur ersten Richtung bewegbar.

Die erste Richtung ist insbesondere parallel zur Längsachse eines mit der Handhabungseinrichtung zu verbindenden Außenschafts bzw. zur Längsachse an dessen proximalem Ende. Wenn die erste und die zweite Richtung senkrecht zueinander sind, kann eine auf den Schlitten und auf den Riegel wirkende Kraft parallel zur ersten Richtung, wie sie beispielsweise zur Verschiebung der zweiten Übertragungseinrichtung im Außenschaft erforderlich ist, keine Bewegung des Riegels relativ zum Schlitten und damit auch keine Entkopplung bewirken.

Bei einer Handhabungseinrichtung, wie sie hier beschrieben ist, ist die zweite Kopplungseinrichtung insbesondere mittels der zweiten Betätigungseinrichtung entlang eines gekrümmten Pfads bewegbar.

Der gekrümmte Pfad, entlang dessen die zweite Betätigungseinrichtung bewegbar ist, ist insbesondere ein kreisbogenförmiger Pfad, dessen Mittelpunkt durch die Schwenkachse der zweiten Betätigungseinrichtung definiert ist, wobei die zweite Kopplungseinrichtung beispielsweise starr mit der zweiten Betätigungseinrichtung verbunden oder sogar zumindest teilweise einstückig mit ihr ausgebildet ist. Insbesondere die starre Verbindung der zweiten Kopplungseinrichtung mit der schwenkbaren zweiten Betätigungseinrichtung kann konstruktiv und fertigungstechnisch besonders einfach realisiert sein.

Die Entkopplungsposition der zweiten Kopplungseinrichtung ist insbesondere am distalen Ende des vorbestimmten Pfads des Schlittens. Die zweite Kopplungseinrichtung kann so ausgebildet sein, dass die zweite Übertragungseinrichtung und die zweite Kopplungseinrichtung in jeder Position der Kopplungseinrichtung gekoppelt werden können.

Bei einer Handhabungseinrichtung, wie sie hier beschrieben ist, ist insbesondere eine formschlüssige Kopplung der zweiten Kopplungseinrichtung mit der zweiten Übertragungseinrichtung von einem Abstand zwischen der zweiten Kopplungseinrichtung und einer Längsachse der zweiten Übertragungseinrichtung abhängig, wobei die Entkopplungsposition der zweiten Kopplungseinrichtung auf dem gekrümmten Pfad so weit von der Längsachse entfernt ist, dass keine Kopplung zwischen der zweiten Kopplungseinrichtung und der zweiten Übertragungseinrichtung vorliegt und andere Positionen der zweiten Kopplungseinrichtung so nahe an der Längsachse liegen, dass eine formschlüssige Kopplung der zweiten Kopplungseinrichtung mit der zweiten Übertragungseinrichtung vorliegen kann.

Andere Positionen der zweiten Kopplungseinrichtung, in denen eine formschlüssige Kopplung der zweiten Kopplungseinrichtung mit der zweiten Übertragungseinrichtung vorliegen kann, werden auch als Arbeitspositionen bezeichnet. Auch in Arbeitspositionen muss keine Kopplung vorliegen, da die zweite Kopplungseinrichtung diese Arbeitspositionen insbesondere auch einnehmen kann, wenn die Handhabungseinrichtung nicht mit einem Außenschaft und Übertragungseinrichtungen in einem Außenschaft verbunden ist.

Bei einer Handhabungseinrichtung, wie sie hier beschrieben ist, umfasst die zweite Kopplungseinrichtung insbesondere einen Riegel, wobei der Riegel durch Betätigung der zweiten Betätigungseinrichtung entlang des gekrümmten Pfads verschiebbar ist, und wobei der Riegel relativ zur zweiten Betätigungseinrichtung entlang eines weiteren Pfads bewegbar ist, der im Wesentlichen senkrecht zu dem gekrümmten Pfad ist.

Die mechanische Kopplung zwischen der zweiten Kopplungseinrichtung und der zweiten Übertragungseinrichtung erfolgt insbesondere mittels des Riegels. Der weitere Pfad ist insbesondere gerade. Der weitere Pfad ist insbesondere dann im Wesentlichen senkrecht zu dem gekrümmten Pfad, wenn der Winkel zwischen dem gekrümmten Pfad und dem weiteren Pfad mindestens 60° oder mindestens 80° beträgt. Die Kopplung der zweiten Kopplungseinrichtung mit der zweiten Übertragungseinrichtung erfolgt insbesondere durch formschlüssige Kopplung von Zapfen an der zweiten Übertragungseinrichtung mit dem Riegel, beispielsweise durch Aufnahme von Zapfen an der zweiten Übertragungseinrichtung in korrespondierenden Nuten am Riegel. Die zweite Übertragungseinrichtung und der Riegel sind insbesondere parallel zu dem weiteren Pfad relativ zueinander verschiebbar, beispielsweise indem die genannten Nuten am Riegel parallel zu dem weiteren Pfad sind.

Bei einer Handhabungseinrichtung, wie sie hier beschrieben ist, ist der Riegel insbesondere relativ zu der zweiten Betätigungseinrichtung entlang des weiteren Pfads gegen eine elastische Kraft eines elastischen Elements aus einer Arbeitsposition heraus bewegbar, wobei der Riegel eine Gleitfläche aufweist, die gegenüber dem gekrümmten Pfad und gegenüber dem weiteren Pfad geneigt und so ausgebildet und angeordnet ist, dass ein in die Handhabungseinrichtung eingeführtes proximales Ende einer zweiten Übertragungseinrichtung an der Gleitfläche gleitend den Riegel entlang des weiteren Pfads verschieben kann.

Die Gleitfläche mit den genannten Eigenschaften kann ein Verschieben des Riegels durch einfaches Einführen des proximalen Endes der zweiten Übertragungseinrichtung in die Handhabungseinrichtung ermöglichen. Dabei kann der Riegel eine Kopplungsposition erreichen, in der die zweite Übertragungseinrichtung mit der zweiten Kopplungseinrichtung gekoppelt wird, beispielsweise indem der Riegel angetrieben durch das elastische Element von der Kopplungsposition wieder in die Arbeitsposition übergeht. Die Gleitfläche mit den beschriebenen Eigenschaften kann eine Kopplung einer zweiten Übertragungseinrichtung mit der zweiten Kopplungseinrichtung in Arbeitspositionen der zweiten Kopplungseinrichtung ermöglichen.

Bei einer Handhabungseinrichtung, wie sie hier beschrieben ist, ist die zweite Kopplungseinrichtung insbesondere so ausgebildet, dass die zweite Übertragungseinrichtung relativ zur zweiten Kopplungseinrichtung um ihre Längsachse rotierbar ist.

Eine Handhabungseinrichtung, wie sie hier beschrieben ist, kann eine Feder oder ein anderes elastisches Element aufweisen, das derart mit der zweiten Betätigungseinrichtung gekoppelt ist, dass es die zweite Betätigungseinrichtung in eine vorbestimmte Position bewegt.

Ein medizinisches Instrument weist eine Handhabungseinrichtung, wie sie hier beschrieben ist, und einen Schaft, der einen Außenschaft und in dem Außenschaft eine erste Übertragungseinrichtung und eine zweite Übertragungseinrichtung umfasst, auf.

Bei einem medizinischen Instrument, wie es hier beschrieben ist, verhindert das proximale Ende des Außenschafts, dass die zweite Kopplungseinrichtung ihre Entkopplungsposition erreicht, wenn der Außenschaft mit dem Grundkörper in der vorgesehenen Weise verbunden ist.

Der Außenschaft ist mit dem Grundkörper insbesondere dann in der vorgesehenen Weise verbunden, wenn er in ihm verriegelt ist.

Bei einem medizinischen Instrument, wie es hier beschrieben ist, umfasst die zweite Übertragungseinrichtung insbesondere einen Innenschaft, wobei das proximale Ende des Innenschafts von einem metallischen Bauelement gebildet wird, und wobei das metallische Bauelement von einem mittleren Abschnitt des Innenschafts elektrisch isoliert ist.

Bei einem medizinischen Instrument, wie es hier beschrieben ist, umfassen insbesondere die zweite Übertragungseinrichtung einen Innenschaft in dem Außenschaft und die erste Übertragungseinrichtung einen Übertragungsstange in dem Innenschaft, wobei der Innenschaft und die Übertragungsstange so ausgebildet sind, dass ein elektrisch leitfähiges proximales Ende des Innenschafts in jeder Position zumindest entweder von dem Außenschaft oder von der Übertragungsstange elektrisch isoliert ist.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine schematische Schnittdarstellung einer Handhabungseinrichtung;
- Figur 3: eine weitere schematische Schnittdarstellung der Handhabungseinrichtung aus Figur 2;
- Figur 4: eine weitere schematische Schnittdarstellung der Handhabungseinrichtung aus den Figuren 2 und 3;
- Figur 5: eine weitere schematische Schnittdarstellung der Handhabungseinrichtung aus den Figuren 2 bis 4;
- Figur 6: eine schematische Schnittdarstellung einer weiteren Handhabungseinrichtung;
- Figur 7: eine weitere schematische Schnittdarstellung der Handhabungseinrichtung aus Figur 6;
- Figur 8: eine weitere schematische Schnittdarstellung der Handhabungseinrichtung aus den Figuren 6 und 7;
- Figur 9: eine weitere schematische Schnittdarstellung der Handhabungseinrichtung aus den Figuren 6 bis 8.

### Beschreibung der Ausführungsformen

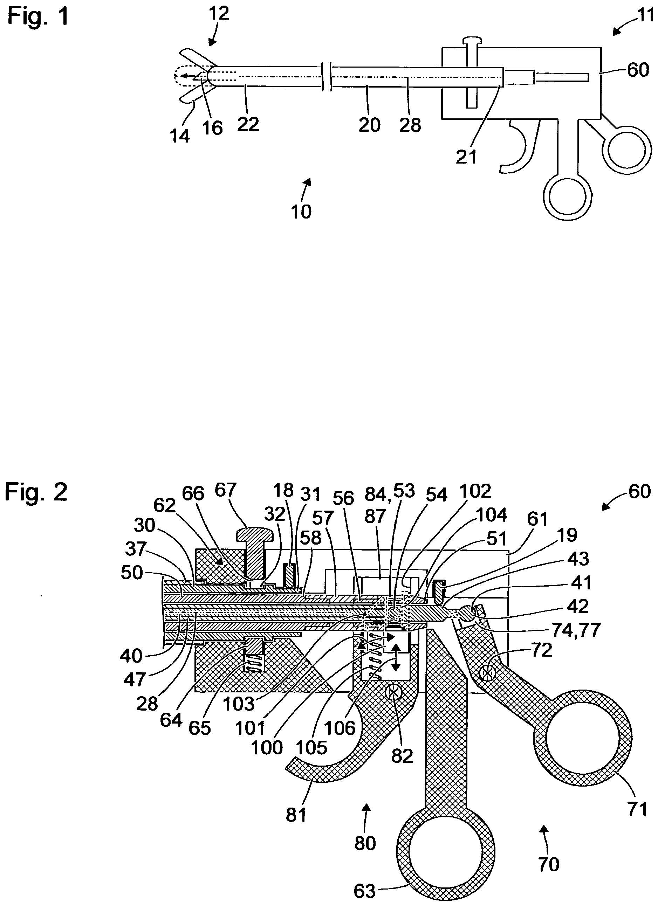

Figur 1 zeigt eine schematische Darstellung eines medizinischen Instruments 10 mit einem proximalen Ende 11 und einem distalen Ende 12. Am distalen Ende 12 weist das medizinische Instrument 10 ein erstes Werkzeug 14 und ein zweiten Werkzeug 16 auf. Das erste Werkzeug 14 und das zweite Werkzeug 16 können unabhängig voneinander bewegt und verwendet werden. Bei dem dargestellten Beispiel umfasst das erste Werkzeug 14 zwei symmetrisch zueinander um Schwenkachsen senkrecht zur Zeichenebene der Figur 1 schwenkbare Maulteile. Das zweite Werkzeug 16 ist bei dem dargestellten Beispiel ein Skalpell, das, wie durch einen Pfeil in Figur 1 angedeutet ist, zwischen den beiden Maulteilen des ersten Werkzeugs 14 bewegbar ist. Die Maulteile des ersten Werkzeugs 14 sind insbesondere so ausgebildet, dass auch in ihren in Figur 1 durch gestrichelte Linien angedeuteten geschlossenen Positionen zwischen den Maulteilen des ersten Werkzeugs 14 ein Kanal bzw. ein Hohlraum verbleibt, in dem das zweite Werkzeug verschiebbar ist.

Das erste Werkzeug 14 ist insbesondere als bipolares elektrochirurgisches Werkzeug ausgebildet. Nach dem Fassen und Quetschen von Gewebe durch die Maulteile des ersten Werkzeugs 14 kann eine hochfrequente Wechselspannung zwischen den Maulteilen des ersten Werkzeugs 14 angelegt werden. Dadurch kann das Gewebe verödet werden. Danach kann das Gewebe mittels des zweiten Werkzeugs 16 durchtrennt werden.

Das medizinische Instrument 10 weist an seinem proximalen Ende 11 eine Handhabungseinrichtung mit mehreren Betätigungseinrichtungen auf. Ausführungsbeispiele der Handhabungseinrichtung 60 sind unten mit Bezug auf die Figuren 2 bis 9 dargestellt.

Ein Schaft 20 verbindet die Handhabungseinrichtung 60 am proximalen Ende 11 des medizinischen Instruments 10 mit den Werkzeugen 14, 16 am distalen Ende 12. Der Schaft 20 ist gerade oder - abweichend von der Darstellung in Figur 1 - gekrümmt, starr oder flexibel. Der Schaft 20 weist eine Längsachse 28 auf, die insbesondere die Symmetrieachse der äußeren Oberfläche des Schafts 20 ist. Im Falle eines gekrümmten Schafts 20 ist nachfolgend mit der Längsachse 28 die Längsachse des Schafts 20 nahe seinem proximalen Ende 21 gemeint.

Das proximale Ende 21 des Schafts 20 ist mit der Handhabungseinrichtung 60 verbunden, insbesondere in einer nachfolgend als Kupplung bezeichneten Ausnehmung korrespondierender Gestalt angeordnet und dort verriegelt bzw. formschlüssig gehalten. Das distale Ende 22 des Schafts 20 ist mit dem ersten Werkzeug 14 und dem zweiten Werkzeug 16 insbesondere lösbar verbunden.

Figur 2 zeigt eine schematische Darstellung eines Schnitts durch ein Ausführungsbeispiel einer Handhabungseinrichtung 60. Die dargestellte Schnittebene ist parallel zur Längsachse 28 und zur Zeichenebene der Figur 1. Die Handhabungseinrichtung 60 kann ausgebildet sein, um mit einem Schaft und Werkzeugen ein medizinisches Instrument zu bilden, das die oben anhand der Figur 1 dargestellten Merkmale oder andere Merkmale aufweist.

Die Handhabungseinrichtung 60 weist einen Grundkörper 61 auf, in dem eine Kupplung 62 für das proximale Ende 31 eines Außenschafts 30 vorgesehen ist. Die Kupplung 62 umfasst eine Ausnehmung mit zum proximalen Ende 31 des Außenschafts 30 korrespondierender Gestalt, so dass der Außenschaft von distal in die Ausnehmung eingeführt werden kann. Insbesondere sind sowohl das proximale Ende 31 des Außenschafts 30 als auch die korrespondierende Ausnehmung im Grundkörper 61 im Wesentlichen rotationssymmetrisch zur Längsachse 28.

Der Außenschaft weist nahe seinem proximalen Ende 31 eine umlaufende Nut 32 auf. Die Kupplung 62 an der Handhabungseinrichtung 60 umfasst einen Riegel 64, der in einer korrespondierenden Ausnehmung im Grundkörper 61 in einer Richtung senkrecht zur Längsachse 28 des Außenschafts 30 und parallel zur Schnittebene der Figur 2 bewegbar ist. Eine Feder 65 schiebt den Riegel 64 in die in Figur 2 dargestellte Position. Der Riegel 64 weist eine Durchgangsbohrung 66 auf, deren Querschnitt im Wesentlichen dem Querschnitt des Außenschafts 30 entspricht. An einem von der Feder 65 abgewandten Ende weist der Riegel 64 einen Druckknopf 67 auf, der aus dem Grundkörper 61 der Handhabungseinrichtung 60 herausragt.

In der in Figur 2 dargestellten verriegelnden Position des Riegels 64 greift der Riegel 64, genauer: der Rand der Durchgangsbohrung 66 im Riegel 64 in die Nut 32 am Außenschaft 30 ein. Dadurch ist der Außenschaft 30 formschlüssig in der in Figur 2 dargestellten Position gehalten, und die mechanische Verbindung zwischen der Handhabungseinrichtung 60 und dem Außenschaft 30 ist durch den Riegel 64 verriegelt. Durch manuellen Druck auf den Druckknopf 67 kann der Riegel 64 gegen die Kraft der Feder 65 in eine entriegelnde Position verschoben werden, in der das proximale Ende 31 des Außenschafts 30 aus der Handhabungseinrichtung 60 entnommen werden kann.

Im Außenschaft 30 sind koaxial eine Übertragungsstange 40 und ein Innenschaft 50 angeordnet. Die Übertragungsstange 40 weist an ihrem proximalen Ende 41 eine Kugel 42 auf. An die Kugel 42 schließt sich nach distal ein Kontaktbereich 43 mit metallischer Oberfläche an. Der Kontaktbereich 43 und die Kugel 42 sind durch einen Hals miteinander verbunden. Distal des Kontaktbereichs 43 weist die Übertragungsstange 40 einen Isoliermantel 47 auf, der die Übertragungsstange 43 nach außen isoliert.

Der Innenschaft 50 ist im Wesentlichen rohrförmig und in dem ringförmigen Zwischenraum zwischen der Übertragungsstange 40 und dem Außenschaft 30 angeordnet. Der Innenschaft 50 umfasst an seinem proximalen Ende 51 einen Ring 53 mit zwei einander gegenüberliegenden Zapfen 54, von denen in der Darstellung in Figur 2 je einer an der dem Betrachter zugewandten und an der vom Betrachter abgewandten Seite des Rings 53 angeordnet ist. Beide Zapfen 54 am Ring 53 liegen somit außerhalb der in Figur 2 gezeigten Schnittebene, sind jedoch durch eine gestrichelte kreisförmige Kontur angedeutet.

Der Ring 53 ist in einer korrespondierenden Nut am proximalen Ende 51 des Innenschafts 50 spiel- und reibungsarm um die Längsachse 28 rotierbar gelagert. Das proximale Ende 51 des Innenschafts mit der den Ring 53 aufnehmenden Nut wird durch ein metallisches Bauteil 56 gebildet, das über einen Isolator 57 mit einem ebenfalls metallischen mittleren Abschnitt 58 des Innenschafts 50 mechanisch verbunden und von diesem elektrisch isoliert ist.

Die Handhabungseinrichtung 60 umfasst ein feststehendes Griffteil 63, das insbesondere einstückig mit dem Grundkörper 61 ausgebildet ist. Ferner umfasst die Handhabungseinrichtung 60 eine hebelartige erste Betätigungseinrichtung 70 mit einem ersten Griffteil 71, die um eine erste Schwenkachse 72 senkrecht zur Zeichenebene der Figur 2 schwenkbar am Grundkörper 61 gelagert ist. Die erste Betätigungseinrichtung 70, insbesondere das erste Griffteil 71 der ersten Betätigungseinrichtung 70, ist proximal des feststehenden Griffteils 63 angeordnet.

Eine erste Kopplungseinrichtung 74 zur Kopplung der ersten Betätigungseinrichtung 70 mit der Übertragungsstange 40 ist an einem bezogen auf die erste Schwenkachse 72 zum ersten Griffteil 71 entgegengesetzten Ende der ersten Betätigungseinrichtung 70 angeordnet. Eine Bewegung des ersten Griffteils 71 nach distal zum feststehenden Griffteil 63 der Handhabungseinrichtung 60 hin geht deshalb mit einer Bewegung der ersten Kopplungseinrichtung 74 nach proximal einher.

Die erste Kopplungseinrichtung 74 ist ausgebildet, um die Kugel 42 am proximalen Ende 41 der Übertragungsstange 40 in der in Figur 2 gezeigten Arbeitsposition und in weiteren, weiter proximal liegenden Arbeitspositionen formschlüssig zu halten. Insbesondere weist die erste Kopplungseinrichtung 74 in einer Schnittebene parallel zur Längsachse 28 und parallel zur ersten Schwenkachse 72 im Wesentlichen die Gestalt eines Hufeisens oder eines großen griechischen Omegas auf, wobei die beiden Enden des Querschnitts unmittelbar distal der Kugel 42 angeordnet sind. Die Kugel 42 wird somit auch an ihrer distalen Seite von der ersten Kopplungseinrichtung 74 teilweise umgriffen und kann von der ersten Kopplungseinrichtung 74 nach proximal gezogen werden.

Die in Figur 2 gezeigte Position der erste Kopplungseinrichtung 74 wird nachfolgend auch als erste Arbeitsposition 77 bezeichnet. Durch Merkmale oder Einrichtungen der Handhabungseinrichtung 60, die in Figur 2 nicht dargestellt sind, kann sichergestellt sein, dass die in Figur 2 gezeigte erste Arbeitsposition 77 die äußerst distale Position der ersten Kopplungseinrichtung 74 ist, die erste Kopplungseinrichtung 74 also nicht weiter nach distal geschwenkt werden kann.

Ferner umfasst die Handhabungseinrichtung 60 eine hebelartige zweite Betätigungseinrichtung 80, die distal des feststehenden Griffteils 63 angeordnet ist. Die zweite Betätigungseinrichtung 80 umfasst ein zweites Griffteil 81 und ist um eine zweite Schwenkachse 82 senkrecht zur Zeichenebene der Figur 2 schwenkbar. Die zweite Betätigungseinrichtung 80 umfasst eine zweite Kopplungseinrichtung 84 zur lösbaren mechanischen Kopplung der zweiten Betätigungseinrichtung 80 mit dem proximalen Ende 51 des Innenschafts 50. Bezogen auf die zweite Schwenkachse 82 der zweiten Betätigungseinrichtung 80 ist die zweite Kopplungseinrichtung 84 an dem zum zweiten Griffteil 81 entgegengesetzten Ende der zweiten Betätigungseinrichtung 80 angeordnet.

Bei einem Schwenken der zweiten Betätigungseinrichtung 80 um die zweite Schwenkachse 82 wird die zweite Kopplungseinrichtung 84 auf einem kreisbogenförmigen Pfad 101 bewegt. Bei der in Figur 2 gezeigten Position der zweiten Betätigungseinrichtung 80 nimmt die zweite Kopplungseinrichtung eine erste Arbeitsposition 87 ein. Die erste Arbeitsposition ist die äußerst proximale Position der zweiten Kopplungseinrichtung 84.

Die zweite Kopplungseinrichtung 84 umfasst einen Riegel 100. Bei einem Schwenken der zweiten Betätigungseinrichtung 80 um die zweite Schwenkachse 82 wird der Riegel 100 zusammen mit der zweiten Kopplungseinrichtung 84 auf dem kreisbogenförmigen Pfad 101 bewegt. Ferner ist der Riegel 100 relativ zur zweiten Kopplungseinrichtung entlang eines Pfads 106 senkrecht zur zweiten Schwenkachse 82 bewegbar. Der Pfad 106 ist durch eine formschlüssige Linearführung 102 des Riegels 100 in der zweiten Betätigungseinrichtung 80 definiert. Wenn die zweite Betätigungseinrichtung 80 um die zweite Schwenkachse 82 geschwenkt wird, wird auch der Pfad 106 entsprechend verschwenkt, entlang dessen der Riegel 100 relativ zur zweiten Betätigungseinrichtung 80 bewegbar ist. Bei der in Figur 2 dargestellten Position der zweiten Betätigungseinrichtung 80 ist der Pfad 106 im Wesentlichen senkrecht zur Längsachse 28.

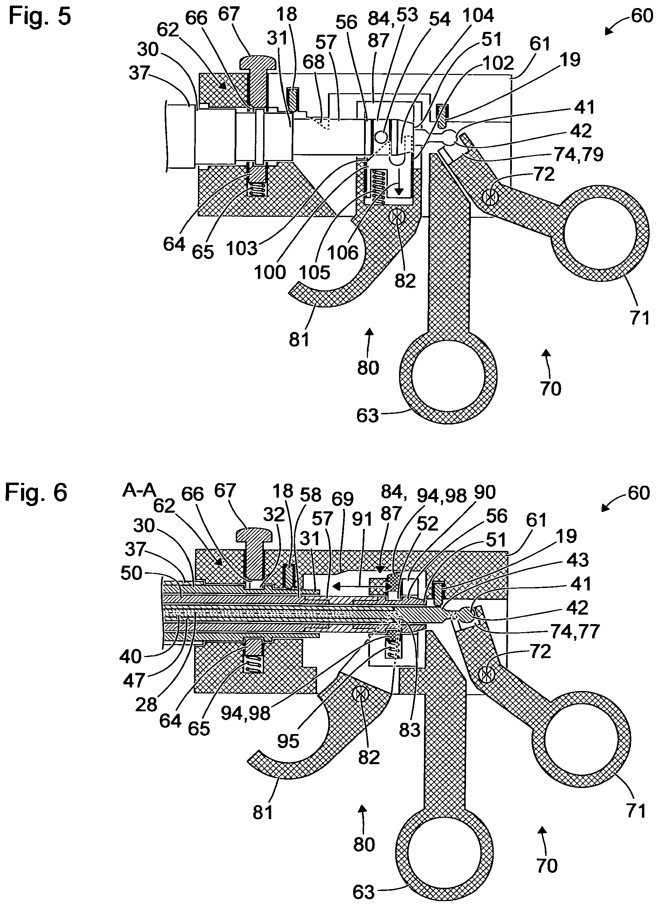

Der Riegel 100 weist zwei Gleitflächen 103 auf, von denen eine vor und eine hinter der Zeichenebene der Figur 2 liegt. Die Gleitflächen 103 sind gegenüber dem Pfad 106, entlang dessen der Riegel 100 relativ zur zweiten Betätigungseinrichtung 80 bewegbar ist, geneigt. Insbesondere beträgt der Winkel zwischen den Gleitflächen 103 und dem Pfad 106 zwischen 20° und 70°. Wie nachfolgend anhand der Figur 5 dargestellt wird, sind die Gleitflächen 103 vorgesehen und ausgebildet, um an den Zapfen 54 am Ring 53 am proximalen Ende 51 des Innenschafts 50 zu gleiten.

Ferner weist der Riegel 100 zwei Nuten 104 zur Aufnahme je eines Zapfens 54 am Ring 53 am proximalen Ende 51 des Innenschafts 50 auf. Je eine Nut 104 liegt vor und hinter der Schnittebene der Figur 2. Bei der Darstellung in Figur 2 nimmt jede Nut 104 einen der beiden Zapfen 54 auf. Die Nuten 104 sind parallel oder im Wesentlichen parallel zu dem Pfad 106, entlang dessen der Riegel 100 relativ zur zweiten Betätigungseinrichtung 80 bewegbar ist.

Eine Feder 105 hält den Riegel 100 in der in Figur 2 gezeigten Position relativ zur zweiten Betätigungseinrichtung 80. Gegen die Kraft der Feder 105 kann der Riegel 100 entlang des Pfads 106 zur zweiten Schwenkachse 82 hin bewegt werden.

Die Handhabungseinrichtung 60 umfasst ferner eine erste Kontakteinrichtung 18 und eine zweite Kontakteinrichtung 19, die in Figur 2 jeweils als Stifte mit abgerundeten Enden dargestellt sind. Die erste Kontakteinrichtung 18 ist so angeordnet, dass sie bei der in Figur 2 gezeigten Position des Außenschafts 30 im Bereich von dessen proximalem Ende 31 an dessen äußerer Mantelfläche anliegt und damit in elektrisch leitfähiger Verbindung mit dem Außenschaft 30 steht. Die zweite Kontakteinrichtung 19 ist so angeordnet, dass sie bei der in Figur 2 gezeigten Position der Übertragungsstange 40 an dem Kontaktbereich 43 am proximalen Ende 41 der Übertragungsstange 40 anliegt und mit diesem in elektrisch leitfähigem Kontakt steht.

Die erste Kontakteinrichtung 18 und die zweite Kontakteinrichtung 19 können jeweils durch in Figur 2 nicht dargestellte Federn oder andere elastische Elemente gegen das proximale Ende 31 des Außenschafts 30 bzw. gegen den Kontaktbereich 43 am proximalen Ende 41 der Übertragungsstange 40 gedrückt werden.

Die erste Kontakteinrichtung 18 und die zweite Kontakteinrichtung 19 sind voneinander elektrisch isoliert, beispielsweise durch ein elektrisch isolierendes Material des Grundkörpers 61 der Handhabungseinrichtung 60. Die erste Kontakteinrichtung 18 und die zweite Kontakteinrichtung 19 sind jeweils über eine in Figur 2 nicht dargestellte elektrische Leitung elektrisch leitfähig mit je einem Pol einer Hochfrequenz-Hochspannungsquelle verbindbar. Die von der hochfrequenten Hochspannungsquelle bereitgestellten Potenziale werden über die erste Kontakteinrichtung 18 und den Außenschaft 30 einerseits und über die zweite Kontakteinrichtung 19 und die Übertragungsstange 40 andererseits zu zwei Elektroden am Werkzeug 14 (vgl. Figur 1), beispielsweise zu den beiden in Figur 1 angedeuteten Maulteilen, übertragen.

Der Isoliermantel 37 am Außenschaft 30 bewirkt eine elektrische Isolation von der Umwelt, insbesondere von mit dem Außenschaft 30 berührten Gegenständen. Der Isoliermantel 47 an der Übertragungsstange 40 stellt eine elektrische Isolation der Übertragungsstange 40 vom elektrisch leitfähigen mittleren Abschnitt 58 des Innenschafts und vom Außenschaft 30 sicher. Das metallische Bauteil 56 am proximalen Ende 51 des Innenschafts 50, das bei den in Figur 2 dargestellten Positionen des Innenschafts 50 und der Übertragungsstange 40 den Kontaktbereich 43 der Übertragungsstange 40 berührt, ist vom mittleren Abschnitt 58 des Innenschafts 50 und damit auch vom Außenschaft 30 durch den Isolator 57 elektrisch isoliert.

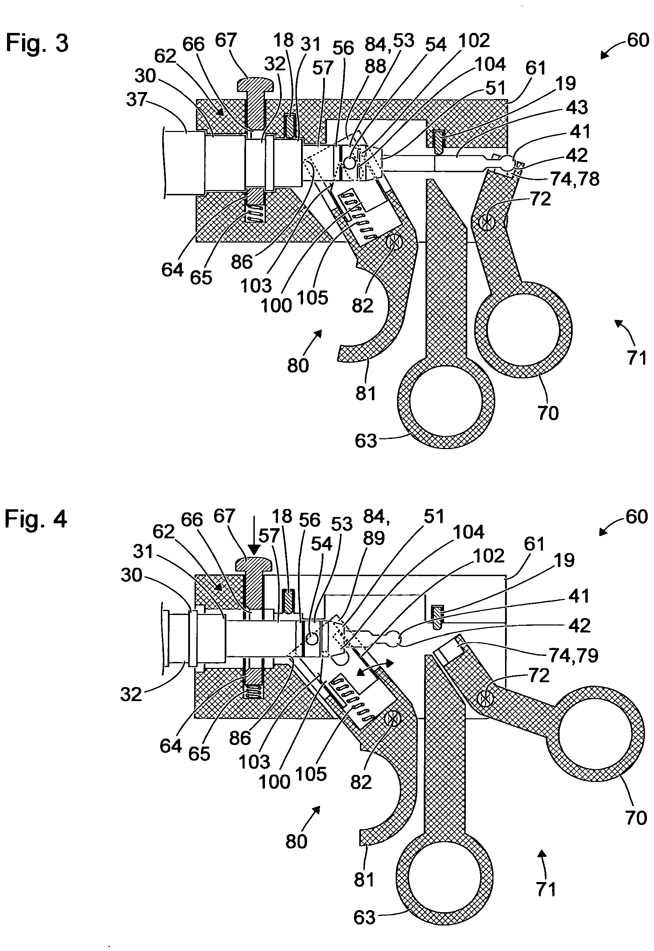

Figur 3 zeigt eine weitere schematische Schnittdarstellung der Handhabungseinrichtung 60 aus Figur 2. Die Darstellung in Figur 3 unterscheidet sich von der Darstellung in Figur 2 dadurch, dass lediglich die Handhabungseinrichtung 60 selbst im Schnitt dargestellt ist. Der Außenschaft 30, der Innenschaft 50 und die Übertragungsstange 40 sind hingegen in einer entsprechenden seitlichen Ansicht gezeigt und damit auch deutlich von den Bestandteilen der Handhabungseinrichtung 60 unterscheidbar. In Figur 3 sind die Nut 32 am proximalen Ende 31 des Außenschafts 30, in die der Riegel 64 bzw. der nahe der Feder 65 liegende Bereich des Rands der Durchgangsbohrung 66 eingreift, gut erkennbar. Ferner ist der Ring 53 mit dem dem Betrachter zugewandten Zapfen 54 an proximalen Ende 51 des Innenschafts 50 gut erkennbar.

Die Darstellung in Figur 3 unterscheidet sich von der Darstellung in Figur 2 ferner dadurch, dass die Übertragungsstange 40, die Innenschaft 50, die erste Betätigungseinrichtung 70 und die zweite Betätigungseinrichtung 80 andere Positionen einnehmen. Insbesondere sind das erste Griffteil 71 der ersten Betätigungseinrichtung 70 nach distal und entsprechend die erste Kopplungseinrichtung 74 nach proximal in eine zweite Arbeitsposition 78 geschwenkt und die Übertragungsstange 40 nach proximal verschoben. Die in Figur 3 dargestellte äußerst proximale Position der Übertragungsstange 40 entspricht beispielsweise der geschlossenen Position von einem oder zwei schwenkbaren Maulteilen eines Werkzeugs am distalen Ende des Schafts. Auch in der in Figur 3 dargestellten proximalen Position der Übertragungsstange 40 liegt die zweite Kontakteinrichtung 19 am Kontaktbereich 43 am proximalen Ende 41 der Übertragungsstange 40 an und stellt auf diese Weise eine elektrisch leitfähige Verbindung zur Übertragungsstange 40 her.

Bei der in Figur 3 gezeigten Position der zweiten Betätigungseinrichtung 80 sind das zweite Griffteil 81 nach proximal und entsprechend die zweite Kopplungseinrichtung 84 nach distal in eine zweite Arbeitsposition 88 geschwenkt und der Innenschaft 50 nach distal verschoben. Bei der dargestellten zweiten Arbeitsposition 88 der zweiten Betätigungseinrichtung 80 liegt diese, insbesondere je ein Eck der vom Betrachter aus gesehen vor bzw. hinter dem Innenschaft 50 liegenden Linearführungen 102, an einer proximalen Stirnfläche des proximalen Endes 31 des Außenschafts 30 an. Wenn der Außenschaft 30, wie in den Figuren 2 und 3 gezeigt, durch den Riegel 64 in der vorgesehenen und dargestellten Position relativ zur Handhabungseinrichtung 60 verriegelt und gehalten ist, ist somit die zweite Arbeitsposition 88 der zweiten Kopplungseinrichtung 84 die äußerst distale erreichbare Position. Entsprechend ist die in Figur 3 gezeigte Position des Innenschafts 50 die äußerst distale erreichbare Position. Auch bei dieser äußerst distalen erreichbaren Position des Innenschafts 50 und der korrespondierenden Position der zweiten Betätigungseinrichtung 80 liegen die Zapfen 54 am Ring 53 am proximalen Ende 51 des Innenschafts 50 innerhalb der Nut 104 am Riegel 100 an der zweiten Betätigungseinrichtung 80. Dies bedeutet eine formschlüssige Kopplung zwischen der zweiten Betätigungseinrichtung 80 und dem Innenschaft 50.

Figur 4 zeigt eine weitere schematische Schnittdarstellung der Handhabungseinrichtung 60 aus den Figuren 2 und 3. Die Darstellung in Figur 4 entspricht insoweit der Darstellung in Figur 3, als lediglich die Handhabungseinrichtung 60 im Schnitt, der Außenschaft 30, die Übertragungsstange 40 und der Innenschaft 50 hingegen in einer entsprechenden Seitenansicht gezeigt sind.

Die Darstellung in Figur 4 unterscheidet sich von den Darstellungen in den Figuren 2 und 3 dadurch, dass durch eine durch einen Pfeil angedeutete Kraft auf den Druckknopf 67 der Riegel 64 gegen die Kraft der Feder 65 in eine entriegelnde Position verschoben ist, in der er nicht mehr in die Nut 32 am proximalen Ende 31 des Außenschafts 30 eingreift, und der Außenschaft 30, der Innenschaft 50 und die Übertragungsstange 40 nach distal verschoben und teilweise aus der Handhabungseinrichtung 60 herausgezogen sind. Durch die Verschiebung des Außenschafts 30 nach distal bildet dieser nicht mehr wie in Figur 3 erkennbar, einen Anschlag für die zweite Betätigungseinrichtung 80. Die zweite Kopplungseinrichtung 84 kann deshalb weiter nach distal bis zu der in Figur 4 gezeigten Entkopplungsposition 89 schwenken. Bei der Entkopplungsposition 89 der zweiten Betätigungseinrichtung 80 und der zweiten Kopplungseinrichtung 84 sind die Zapfen 54 am Ring 53 am proximalen Ende 51 des Innenschafts 50 nicht mehr in Eingriff mit den Nuten 104 am Riegel 100 an der zweiten Betätigungseinrichtung 80. Deshalb kann auch der Innenschaft 50 nach distal aus der Handhabungseinrichtung 60 herausgezogen werden.

Aufgrund der Verschiebung des Außenschafts 30 nach distal ist auch eine Verschiebung der Übertragungsstange 40 über die in Figur 2 gezeigte Position hinaus nach distal möglich. Dabei erreicht die erste Kopplungseinrichtung 74 die in Figur 4 gezeigte Entkopplungsposition 79, in der die Kugel 42 am proximalen Ende 41 der Übertragungsstange 40 nicht mehr formschlüssig mit der ersten Kopplungseinrichtung 74 an der ersten Betätigungseinrichtung 70 verbunden ist. Die erste Betätigungseinrichtung 70 wird insbesondere durch eine in Figur 4 nicht gezeigte Feder in der in Figur 4 dargestellten Position gehalten.

Die der Schwenkbewegung der zweiten Betätigungseinrichtung 80 entsprechende Bewegung der zweiten Kopplungseinrichtung 84 auf dem kreisbogenförmigen Pfad 101 entspricht einer Verschiebung der zweiten Kopplungseinrichtung 84 parallel zur Längsachse 28, die überlagert ist von einer Bewegung der zweiten Kopplungseinrichtung 84 in Richtung senkrecht zur Längsachse 28. Dadurch variiert der Abstand der zweiten Kopplungseinrichtung 84 von der Längsachse 28 während einer Schwenkbewegung der zweiten Betätigungseinrichtung 80.

Bei den in den Figuren 2 und 3 dargestellten Arbeitspositionen 87, 88 der zweiten Betätigungseinrichtung 80 weist die zweite Kopplungseinrichtung 84 einen bei der in Figur 3 gezeigten Position erreichten maximalen Abstand von der Längsachse 28 auf, bei der die Zapfen 54 am Ring 53 am proximalen Ende 51 des Innenschafts noch immer in Eingriff mit den Nuten 104 am Riegel 100 an der zweiten Betätigungseinrichtung 80 sind. Hingegen ist bei der in Figur 4 gezeigten Entkopplungsposition 89 der zweiten Kopplungseinrichtung 84 der Abstand zwischen der zweiten Kopplungseinrichtung 84 und der Längsachse 28 so groß, dass die Zapfen 54 am Ring 53 am proximalen Ende 51 des Innenschafts 50 nicht mehr in Eingriff mit den Nuten 104 am Riegel 100 der zweiten Betätigungseinrichtung 80 sind.

Figur 5 zeigt eine weitere schematische Schnittdarstellung der Handhabungseinrichtung 60 aus den Figuren 2 bis 4. Die Darstellung in Figur 5 entspricht insoweit den Darstellungen in den Figuren 3 und 4, als lediglich die Handhabungseinrichtung 60 im Schnitt, der Außenschaft 30, der Innenschaft 50 und die Übertragungsstange 40 hingegen in einer entsprechenden Seitenansicht dargestellt sind.

Figur 5 zeigt die Handhabungseinrichtung 60, den Außenschaft 30, den Innenschaft 50 und die Übertragungsstange 40 in Positionen bzw. in einer Konfiguration, wie sie beim Einführen des Außenschafts 30, des Innenschafts 50 und der Übertragungsstange 40 von distal nach proximal in die Handhabungseinrichtung 60 vorübergehend vorliegen kann. Durch die bereits oben in der Beschreibung der Figur 4 erwähnte, in den Figuren 2 bis 5 nicht gezeigte Feder ist die erste Betätigungseinrichtung 70 in der bereits in Figur 4 gezeigten Entkopplungsposition 79 gehalten, in der die erste Kopplungseinrichtung 74 an der ersten Betätigungseinrichtung 70 die Kugel 42 am proximalen Ende 41 der Übertragungsstange 40 aufnehmen kann.

Beim Einführen des Innenschafts 50 in die Handhabungseinrichtung 60 kann der Ring 53 mit den Zapfen 54 am proximalen Ende 51 des Innenschafts 50 bezogen auf die mögliche Rotation um die Längsachse 28 eine beliebige Position einnehmen. Durch eine Ausrichteeinrichtung 68 wird der Ring 53 mit den Zapfen 54 beim Einführen des Innenschafts 50 in die Handhabungseinrichtung 60 in die in Figur 5 gezeigte Position ausgerichtet. Die Ausrichteeinrichtung 68 weist zwei keilförmig bzw. V-förmig angeordnete Gleitflächen oder Gleitkanten auf, die sich entlang der äußeren Mantelfläche des Innenschafts 50 erstrecken, und an denen einer der beiden Zapfen 54 entlang gleitet bis der Ring 53 mit den Zapfen 54 die vorgesehene, in den Figuren 2 bis 5 gezeigte Position einnimmt.

Im Gegensatz zur ersten Betätigungseinrichtung 70 kann die zweite Betätigungseinrichtung 80 beim Einführen des Außenschafts 30 mit dem Innenschaft 50 und der Übertragungsstange 40 eine beliebige Position einnehmen. Wenn die zweite Betätigungseinrichtung 80 zufällig die in Figur 4 gezeigte Entkopplungsposition 89 einnimmt, können die Zapfen 54 am Ring 53 am proximalen Ende 51 des Innenschafts 50 unmittelbar in die Nuten 104 am Riegel 100 an der zweiten Betätigungseinrichtung 80 aufgenommen werden.

Wenn die zweite Betätigungseinrichtung 80 zunächst die in Figur 3 gezeigte zweite Arbeitsposition 88 oder eine Position zwischen den in den Figuren 2 und 3 gezeigten Arbeitspositionen 87, 88 einnimmt, berühren die Zapfen 54 am Ring 53 zunächst die Gleitflächen 103 am Riegel 100. Dadurch wird beim Bewegen des Innenschafts 50 nach proximal zunächst die zweite Kopplungseinrichtung 84 in die in Figur 5 gezeigte äußerst proximale erste Arbeitsposition 87 geschwenkt. Wenn die zweite Kopplungseinrichtung 84 an der zweiten Betätigungseinrichtung 80 die erste Arbeitsposition 87 erreicht hat, bewirkt eine weitere Bewegung des Innenschafts 50 nach proximal aufgrund der Neigung der Gleitflächen 103 am Riegel 100, an denen die Zapfen 54 gleiten, eine Verschiebung des Riegels 100 entlang des Pfads 106 gegen die Kraft der Feder 105 in die in Figur 5 gezeigte Position und noch weiter zur zweiten Schwenkachse 82 hin.

Wenn der Innenschaft 50 von der in Figur 5 gezeigten Position aus noch etwas weiter nach proximal und dadurch der Riegel 100 gegen die Kraft der Feder 105 noch etwas weiter zur zweiten Schwenkachse 82 hin verschoben wird, gelangen die Zapfen 54 in die Nuten 104 im Riegel 100, die Feder 105 bewegt den Riegel 100 von der zweiten Schwenkachse 82 weg in die in Figur 2 gezeigte verriegelnde Position, und die formschlüssige mechanische Kopplung zwischen dem Innenschaft 50 einerseits und dem Riegel 100 und der zweiten Betätigungseinrichtung 80 andererseits ist hergestellt.

Figur 6 zeigt eine schematische Schnittdarstellung einer weiteren Handhabungseinrichtung 60, die in einigen Merkmalen der oben anhand der Figuren 2 bis 5 dargestellten Handhabungseinrichtung ähnelt. Die Darstellung in Figur 6 entspricht insbesondere hinsichtlich der Schnittebene und hinsichtlich der Art der Darstellung des Außenschafts 30, des Innenschafts 50 und der Übertragungsstange 40 der Darstellung in Figur 2.

Die in Figur 6 dargestellte Handhabungseinrichtung 60 unterscheidet sich von der oben anhand der Figuren 2 bis 5 dargestellten Handhabungseinrichtung insbesondere in den Merkmalen der zweiten Kopplungseinrichtung 84. Korrespondierend zu den unterschiedlichen Merkmalen der zweiten Kopplungseinrichtung 84 der Handhabungseinrichtung 60 weist auch das proximale Ende 51 des Innenschafts 50 andere Merkmale auf als bei dem oben anhand der Figuren 2 bis 5 dargestellten Beispiel.

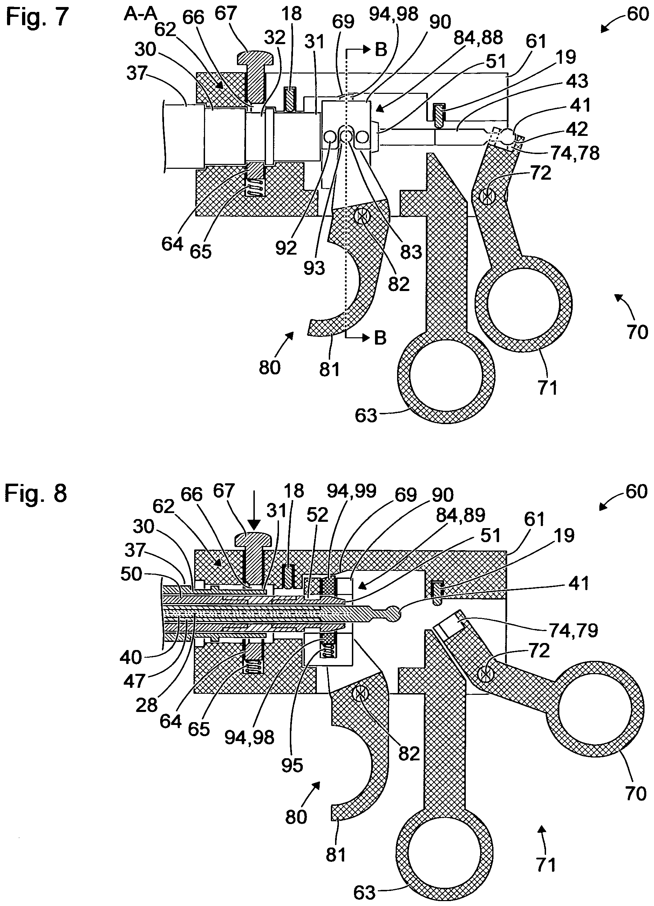

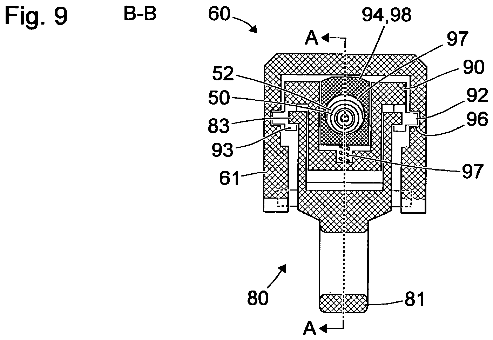

Die zweite Kopplungseinrichtung 84 an der zweiten Betätigungseinrichtung 80 umfasst einen Schlitten 90, der mit der zweiten Betätigungseinrichtung 80 mechanisch gekoppelt, jedoch nicht starr verbunden ist. Der Schlitten 90 ist entlang eines geraden Pfads 91 parallel zur Längsachse 28 bewegbar. Der gerade Pfad 91 ist durch Merkmale des Schlittens 90 und des Grundkörpers 61 der Handhabungseinrichtung 60 definiert, die unten anhand der Figuren 7 und 9 beschrieben sind. Die mechanische Kopplung zwischen der zweiten Betätigungseinrichtung 80 und dem Schlitten 90 der zweiten Kopplungseinrichtung 84 erfolgt durch Merkmale, die ebenfalls unten anhand der Figuren 7 und 9 beschrieben sind. Im Ergebnis geht ein Schwenken der zweiten Betätigungseinrichtung 80 um die zweite Schwenkachse 82 mit einer Bewegung der zweiten Kopplungseinrichtung 84, insbesondere des Schlittens 90, entlang des geraden Pfads 91 einher.

Der Schlitten 90 weist einen Riegel 94 auf, der in einer korrespondierenden Ausnehmung im Schlitten 90 in einer Richtung senkrecht zur Längsachse 28 und senkrecht zum Pfad 91 bewegbar ist. Der Riegel 94 weist insbesondere ähnlich wie der Riegel 64 der Kupplung 62 für den Außenschaft 30 im Wesentlichen die Gestalt einer Platte mit einer Durchgangsbohrung auf, wobei der Querschnitt der Durchgangsbohrung dem Querschnitt des Innenschafts 50 entspricht. Das metallische Bauteil 56 am proximalen Ende 51 des Innenschafts 50 weist ähnlich wie bei der oben anhand der Figuren 2 bis 5 dargestellten Handhabungseinrichtung eine Nut, in der Nut jedoch keinen Ring auf. Eine Feder 95 schiebt den Riegel 94 innerhalb des Schlittens 90 in die in Figur 6 gezeigte Position, in der ein Bereich des Rands der Durchgangsbohrung im Riegel 94 in die Nut 52 am proximalen Ende 51 des Innenschafts 50 eingreift. In dieser Position bildet der in die Nut 52 eingreifende Riegel 94 eine formschlüssige und - von unvermeidlichem Spiel abgesehen - mechanisch starre Verbindung zwischen dem Schlitten 90 bzw. der zweiten Kopplungseinrichtung 84 einerseits und dem proximalen Ende 51 des Innenschafts 50 andererseits.

Figur 6 zeigt die erste Kopplungseinrichtung 74 und die zweite Kopplungsposition 84 in ersten Arbeitspositionen 77, 87. Durch Merkmale oder Einrichtungen der Handhabungseinrichtung 60, die in Figur 6 nicht dargestellt sind, kann sichergestellt sein, dass die in Figur 6 gezeigte erste Arbeitsposition 77 der ersten Kopplungseinrichtung 74 die äußerst distale Position der ersten Kopplungseinrichtung 74 ist, die erste Kopplungseinrichtung 74 also nicht weiter nach distal geschwenkt werden kann. Durch Merkmale oder Einrichtungen der Handhabungseinrichtung 60, die in Figur 2 nicht dargestellt sind, insbesondere durch einen mechanischen Anschlag am Grundkörper 61 für den Schlitten 90 kann sichergestellt sein, dass die in Figur 6 gezeigte erste Arbeitsposition 87 der zweiten Kopplungseinrichtung 84 die äußerst proximale Position der zweiten Kopplungseinrichtung 84 ist, die zweite Kopplungseinrichtung 84 also nicht weiter nach distal geschwenkt werden kann.

Figur 7 zeigt eine weitere schematische Darstellung der Handhabungseinrichtung 60 aus Figur 6, die hinsichtlich der Art der Darstellung derjenigen in Figur 3 ähnelt. Insbesondere sind die Handhabungseinrichtung 60 im Wesentlichen im Schnitt und der Außenschaft 30, der Innenschaft 50 und die Übertragungsstange 40 in einer entsprechenden Seitenansicht gezeigt. Abweichend davon ist jedoch auch die ebenfalls zur Handhabungseinrichtung 60 zählende zweite Kopplungseinrichtung 84, insbesondere der Schlitten 90, nicht im Schnitt, sondern in einer Seitenansicht dargestellt. Die Darstellung in Figur 7 entspricht auch hinsichtlich der Positionen der ersten Betätigungseinrichtung 70, der zweiten Betätigungseinrichtung 80, der Übertragungsstange 40 und des Innenschafts 50 im Wesentlichen der Darstellung in Figur 3. Insbesondere nimmt die erste Kopplungseinrichtung 74 eine zweite Arbeitsposition 78 ein, die die äußerst proximale erreichbare Position der ersten Kopplungseinrichtung 74 ist. Ferner nimmt die zweite Kopplungseinrichtung 84 eine zweite Arbeitspositionen 88 ein, die die äußerst distale erreichbare Position der zweiten Kopplungseinrichtung 84 ist.

In Figur 7 sind zwei Führungszapfen 92 an der dem Betrachter zugewandten Seite des Schlittens 90 sichtbar, die in eine in Figur 7 nicht dargestellte korrespondierende Nut im Grundkörper 61 der Handhabungseinrichtung 60 eingreifen. Symmetrisch zur Schnittebene der Figur 7 greifen zwei weitere, in Figur 7 nicht sichtbare Führungszapfen an der vom Betrachter abgewandten Seite des Schlittens 90 in eine korrespondierende weitere Nut im Grundkörper 61 der Handhabungseinrichtung 60 ein. Die Führungszapfen 92 und die Nuten im Grundkörper 61 der Handhabungseinrichtung 60 definieren den Pfad 91 (vgl. Figur 6), entlang dessen der Schlitten 90 bewegbar ist.

Ein Zapfen 83 an einem vom zweiten Griffteil 81 abgewandten Ende der zweiten Betätigungseinrichtung 80 greift in eine korrespondierende Nut 93 am Schlitten 90 ein. Die Nut 93 am Schlitten 90 erstreckt sich im Wesentlichen senkrecht zum Pfad 91 (vgl. Figur 6), entlang dessen der Schlitten 90 bewegbar ist. Symmetrisch zur Schnittebene der Figur 7 greift ein weiterer Zapfen in eine weitere Nut an der vom Betrachter abgewandten Seite des Schlittens 90 ein. Die Zapfen 83 an der zweiten Betätigungseinrichtung 80 und die Nuten 93 am Schlitten 90 koppeln die zweite Betätigungseinrichtung 80 in der oben beschriebenen Weise mechanisch mit dem Schlitten 90.

Bei der in Figur 7 gezeigten zweiten Arbeitsposition 88 der zweiten Kopplungseinrichtung 84, insbesondere des Schlittens 90, liegt eine distale Stirnfläche des Schlittens 90 an einer proximalen Stirnfläche des proximalen Endes 31 des Außenschafts 30 an. Wenn der Außenschaft 30, wie in den Figuren 6 und 7 dargestellt, in der Handhabungseinrichtung 60 verriegelt ist, stellt somit die in Figur 7 gezeigte zweite Arbeitsposition der zweiten Kopplungseinrichtung 84 und des Schlittens 90 die äußerst distale erreichbare Position dar.

Figur 8 zeigt eine weitere schematische Schnittdarstellung der Handhabungseinrichtung aus den Figuren 6 und 7. Hinsichtlich der Art der Darstellung entspricht die Figur 8 der Figur 6. Insbesondere sind sowohl die Handhabungseinrichtung 60 einschließlich der zweiten Kopplungseinrichtung 84 als auch der Außenschaft 30, der Innenschaft 50 und die Übertragungsstange 40 im Schnitt dargestellt.

Die Darstellung in Figur 8 unterscheidet sich von den Darstellungen in den Figuren 6 und 7 insbesondere dadurch, dass durch eine durch einen Pfeil angedeutete Kraft der Riegel 64 an der Kupplung 62 gegen die Kraft der Feder 65 in die in Figur 8 gezeigte Position verschoben ist, in der er nicht in die Nut 32 am proximalen Ende 31 des Außenschafts 30 eingreift. In dieser entriegelnden Position des Riegels 64 können der Außenschaft 30, der Innenschaft 50 und die Übertragungsstange 40 nach distal aus der Handhabungseinrichtung 60 herausgezogen werden. Figur 8 zeigt beispielhaft dabei vorübergehend eingenommene Positionen des Außenschafts 30, des Innenschafts 50 und der Übertragungsstange 40. Wenn der Außenschaft 30 nicht mehr die in den Figuren 6 und 7 gezeigte Position einnimmt, in der er mit dem Grundkörper 61 der Handhabungseinrichtung 60 mechanisch verbunden und verriegelt ist, kann die zweite Kopplungseinrichtung 84 über die in Figur 7 gezeigte zweite Arbeitsposition 88 hinaus weiter nach distal und bis zu der in Figur 8 gezeigten Position Entkopplungsposition 89 verschoben werden. Dabei wird ein von der Feder 95 abgewandtes Ende des Riegels 94 von einem rampenförmigen Bereich 69 am Grundkörper 61 der Handhabungseinrichtung 60 gegen die Kraft der Feder 95 von einer Arbeitsposition 98 (vgl. Figur 7) in die in Figur 8 gezeigte Entriegelungsposition 99 verschoben, in der der Riegel 94 nicht mehr in die Nut 52 am proximalen Ende 51 des Innenschafts 50 eingreift. Danach kann das proximale Ende 51 des Innenschafts 50, wie in Figur 8 angedeutet, nach distal aus der zweiten Kopplungseinrichtung 84 und insbesondere aus dem Schlitten 90 herausgezogen werden.

Ähnlich wie bei dem Ausführungsbeispiel der Figuren 2 bis 5 ist eine mechanische Verbindung zwischen dem proximalen Ende 51 des Innenschafts 50 und der zweiten Kopplungseinrichtung 84 bzw. dem Schlitten 90 in jeder Position des Schlittens 90 herstellbar. Dabei wird insbesondere der Schlitten 90 zunächst in seine in Figur 6 gezeigte äußerst proximale Position verschoben. Danach bewirkt ein konischer Bereich am proximalen Ende 51 des Innenschafts 50 eine Auslenkung des Riegels 94 gegen die Kraft der Feder 95 bis der Riegel 94 in die Nut 52 am proximalen Ende 51 des Innenschafts 50 eingreift.

Figur 9 zeigt eine weitere schematische Schnittdarstellung der Handhabungseinrichtung aus den Figuren 6 bis 8. Gezeigt ist ein Schnitt entlang der Ebene B-B, die in Figur 7 angedeutet ist. Die Position und Orientierung der Schnittebene A-A der Figuren 6 bis 8 ist in Figur 9 angedeutet. Die Schnittebene B-B ist senkrecht zur Längsachse 28 und senkrecht zur Schnittebene A-A.

In Figur 9 sind eine gabelförmige Gestalt der zweiten Betätigungseinrichtung 80 an ihrem vom zweiten Griffteil 81 beabstandeten Ende, die in Nuten bzw. Schlitze 93 am Schlitten 90 eingreifenden Zapfen 83 an der zweiten Betätigungseinrichtung 80, die Führungszapfen 92 am Schlitten 90, die korrespondierenden Nuten im Grundkörper 61 der Handhabungseinrichtung 60, die plattenförmige Gestalt des Riegels 94 und die Durchgangsbohrung 97 im Riegel 94 für den Innenschaft 50 erkennbar. Der Riegel 94 ist in der Arbeitsposition 98 dargestellt, in der befindet, wenn die zweite Kopplungseinrichtung 84 und der Schlitten 90 sich in einer der Arbeitspositionen 87, 88 befinden.

### Bezugszeichen

- 10: medizinisches Instrument
- 11: proximales Ende des medizinischen Instruments 10
- 12: distales Ende des medizinischen Instruments 10
- 14: erstes Werkzeug am distalen Ende 12 des medizinischen Instruments 10
- 16: zweites Werkzeug am distalen Ende 12 des medizinischen Instruments 10
- 18: erste Kontakteinrichtung
- 19: zweite Kontakteinrichtung
- 20: Schaft des medizinischen Instruments 10
- 21: proximales Ende des Schafts 20
- 22: distales Ende des Schafts 20
- 28: Längsachse des Schafts 20
- 30: Außenschaft
- 31: proximales Ende des Außenschafts 30
- 32: Nut am proximalen Ende 31 des Außenschafts 30
- 37: Isoliermantel am Außenschaft 30
- 40: Übertragungsstange
- 41: proximales Ende der Übertragungsstange 40
- 42: Kugel am proximalen Ende 41 der Übertragungsstange 40
- 43: Kontaktbereich am proximalen Ende 41 der Übertragungsstange 40
- 47: Isoliermantel an der Übertragungsstange 40
- 50: Innenschaft
- 51: proximales Ende des Innenschafts 50
- 52: Nut am proximalen Ende 51 des Innenschafts 50
- 53: Ring am proximalen Ende 51 des Innenschafts 50
- 54: Zapfen am Ring 53
- 56: metallisches Bauteil am proximalen Ende 51 des Innenschafts 50
- 57: Isolator zwischen metallischem Bauteil 56 und mittlerem Abschnitt 58
- 58: mittlerer Abschnitt des Innenschafts 50
- 60: Handhabungseinrichtung am proximalen Ende 11 des medizinischen Instruments 10
- 61: Grundkörper der Handhabungseinrichtung 60
- 62: Kupplung für proximales Ende 31 des Außenschafts 30
- 63: feststehendes Griffteil an der Handhabungseinrichtung 60
- 64: Riegel an der Kupplung 62
- 65: Feder am Riegel 64
- 66: Durchgangsbohrung im Riegel 64
- 67: Druckknopf am Riegel 64
- 68: Ausrichteeinrichtung für Zapfen 54
- 69: rampenförmiger Bereich am Grundkörper 61
- 70: erste Betätigungseinrichtung an der Handhabungseinrichtung 20
- 71: erster Griffteil an der ersten Betätigungseinrichtung 70
- 72: erste Schwenkachse der ersten Betätigungseinrichtung 70
- 74: erste Kopplungseinrichtung zur Kopplung der ersten Betätigungseinrichtung 70 mit der ersten Übertragungseinrichtung 40
- 77: erste Arbeitsposition der ersten Kopplungseinrichtung 74
- 78: zweite Arbeitsposition der ersten Kopplungseinrichtung 74
- 79: Entkopplungsposition der ersten Kopplungseinrichtung 74
- 80: zweite Betätigungseinrichtung an der Handhabungseinrichtung 20
- 81: zweiter Griffteil an der zweiten Betätigungseinrichtung 80
- 82: zweite Schwenkachse der zweiten Betätigungseinrichtung 80
- 83: Zapfen zur mechanischen Kopplung mit dem Schlitten 90
- 84: zweite Kopplungseinrichtung zur Kopplung der zweiten Betätigungseinrichtung 80 mit der zweiten Übertragungseinrichtung 50
- 85: Kopplungsposition bzw. Bereich von Kopplungspositionen
- 86: Ecke der zweiten Betätigungseinrichtung 80
- 87: erste Arbeitsposition der zweiten Kopplungseinrichtung 84
- 88: zweite Arbeitsposition der zweiten Kopplungseinrichtung 84
- 89: Entkopplungsposition der zweiten Kopplungseinrichtung 84
- 90: Schlitten an der zweiten Kopplungseinrichtung 84
- 91: Pfad, entlang dessen der Schlitten 90 bewegbar ist
- 92: Führungszapfen am Schlitten 90
- 93: Nut für den Zapfen 83 an der zweiten Betätigungseinrichtung 80
- 94: Riegel an der zweiten Kopplungseinrichtung 84
- 95: Feder am Riegel 94
- 96: Nut am Grundkörper 61 für den Führungszapfen 92 am Schlitten 90
- 97: Durchgangsbohrung
- 98: Arbeitsposition des Riegels 94
- 99: Entriegelungsposition des Riegels 94
- 100: Riegel an der zweiten Kopplungseinrichtung 84
- 101: Pfad, entlang dessen der Riegel 100 mit der zweiten Betätigungseinrichtung 80 bewegbar ist
- 102: formschlüssige Linearführung des Riegels 100 an der zweiten Betätigungseinrichtung 80
- 103: Gleitfläche am Riegel
- 104: Nut für den Zapfen 54 am Ring 53 am proximalen Ende 53 des Innenschafts 50
- 105: Feder am Riegel 100
- 106: Pfad, entlang dessen der Riegel 100 relativ zur zweiten Betätigungseinrichtung 80 bewegbar ist

## Patentansprüche

1. **Handhabungseinrichtung** (60) für ein medizinisches Instrument (10), mit
einem **Grundkörper** (61) mit einer Kupplung (62) zur lösbaren mechanischen Verbindung mit einem proximalen Ende (31) eines für das medizinische Instrument (10) vorgesehenen Außenschafts (30);
einer ersten **Betätigungseinrichtung** (70), die relativ zum Grundkörper (61) bewegbar ist;
einer zweiten **Betätigungseinrichtung** (80), die relativ zum Grundkörper (61) bewegbar ist;
einer ersten **Kopplungseinrichtung** (74) zur Kopplung der ersten Betätigungseinrichtung (70) mit einer ersten Übertragungseinrichtung (40) zum Übertragen zumindest entweder einer Kraft oder eines Drehmoments;
einer zweiten **Kopplungseinrichtung** (84) zur Kopplung der zweiten Betätigungseinrichtung (80) mit einer zweiten Übertragungseinrichtung (50) zum Übertragen zumindest entweder einer Kraft oder eines Drehmoments;
wobei die erste Kopplungseinrichtung (74) und die zweite Kopplungseinrichtung (84; 90) jeweils eine **Entkopplungsposition** (79, 89) aufweisen, in der die zugeordnete Betätigungseinrichtung (70, 80) von der zugeordneten Übertragungseinrichtung (40, 50) entkoppelt ist,
wobei die Entkopplungspositionen (79, 89) beider Kopplungseinrichtungen (74, 84) nur dann erreichbar sind, wenn der für das medizinische Instrument (10) vorgesehene Außenschaft (30) nicht in der für die Verwendung des medizinischen Instruments vorgesehenen Weise mit der Kupplung (62) am Grundkörper (61) verbunden ist.

2. Handhabungseinrichtung (60) nach dem vorangehenden Anspruch, bei der die zweite Kopplungseinrichtung (84) in jeder Position zumindest entweder von dem proximalen Ende (31) eines für das medizinische Instrument (10) vorgesehenen und mit der Handhabungseinrichtung (60) verbundenen Außenschafts (30) oder von dem proximalen Ende (41) einer mit der ersten Betätigungseinrichtung (70) gekoppelten ersten Übertragungseinrichtung (40) **elektrisch isoliert** ist.

3. Handhabungseinrichtung (60) nach einem der vorangehenden Ansprüche, bei der die erste Betätigungseinrichtung (70) **proximal** eines feststehenden Griffteils (63) der Handhabungseinrichtung (60) und die zweite Betätigungseinrichtung (80) **distal** des feststehenden Griffteils (63) angeordnet sind.

4. Handhabungseinrichtung (60) nach einem der vorangehenden Ansprüche, bei der die zweite Kopplungseinrichtung (84) einen **Schlitten** (90) umfasst, der mittels der zweiten Betätigungseinrichtung (80) entlang eines vorbestimmten Pfads (91) verschiebbar ist.

5. Handhabungseinrichtung (60) nach dem vorangehenden Anspruch, bei der
die zweite Kopplungseinrichtung (84) einen **Riegel** (94) zum formschlüssigen Halten des proximalen Endes der zweiten Übertragungseinrichtung (50) an dem Schlitten (90) umfasst,
der Grundkörper (61) ausgebildet ist, um bei Annäherung der zweiten Kopplungseinrichtung (84) an die Entkopplungsposition (89) den Riegel (94) in eine **Entriegelungsposition** (99) zu bewegen, in der der Riegel (94) das proximale Ende (51) der zweiten Übertragungseinrichtung (50) nicht mehr formschlüssig hält.

6. Handhabungseinrichtung (60) nach dem vorangehenden Anspruch, bei der
der Schlitten (90) in einer ersten Richtung (91) bewegbar ist,
der **Riegel** (94) relativ zu dem Schlitten (90) in einer zweiten Richtung (92) **senkrecht** zur ersten Richtung (91) **bewegbar** ist.

7. Handhabungseinrichtung (60) nach einem der Ansprüche 1 bis 4, bei der die zweite Kopplungseinrichtung (84) mittels der zweiten Betätigungseinrichtung (80) entlang eines gekrümmten Pfads (101) bewegbar ist.

8. Handhabungseinrichtung (60) nach dem vorangehenden Anspruch, bei der
eine formschlüssige **Kopplung** der zweiten Kopplungseinrichtung (84) mit der zweiten Übertragungseinrichtung (50) von einem **Abstand** zwischen der zweiten Kopplungseinrichtung (84) und **einer Längsachse** (28) der zweiten Übertragungseinrichtung (50) abhängig ist,
die **Entkopplungsposition** (89) der zweiten Kopplungseinrichtung (84) auf dem gekrümmten Pfad (101) so **weit** von der Längsachse (28) **entfernt** ist, dass keine Kopplung zwischen der zweiten Kopplungseinrichtung (84) und der zweiten Übertragungseinrichtung vorliegt,
andere Positionen (87, 88) der zweiten Kopplungseinrichtung (84) so nah an der Längsachse (28) liegen, dass eine formschlüssige Kopplung der zweiten Kopplungseinrichtung (84) mit der zweiten Übertragungseinrichtung (50) vorliegen kann.

9. Handhabungseinrichtung (60) nach einem der vorangehenden Ansprüche, bei der die zweite Kopplungseinrichtung (84) einen **Riegel** (100) umfasst,
wobei der Riegel (100) durch Betätigung der zweiten Betätigungseinrichtung (80) entlang des gekrümmten Pfad (101) verschiebbar ist,
wobei der Riegel (100) relativ zur zweiten Betätigungseinrichtung (80) entlang eines weiteren Pfads (106) bewegbar ist, der im Wesentlichen **senkrecht** zu dem gekrümmten Pfad (101) ist.

10. Handhabungseinrichtung (60) nach dem vorangehenden Anspruch, bei der
der Riegel (105) relativ zu der zweiten Betätigungseinrichtung (80) entlang des weiteren Pfads (106) gegen eine elastische Kraft eines **elastischen Elements** (105) aus einer Arbeitsposition (108) heraus bewegbar ist,
der Riegel (100) eine **Gleitfläche** (103) aufweist, die gegenüber dem gekrümmten Pfad (101) und gegenüber dem weiteren Pfad (106) geneigt und so ausgebildet und angeordnet ist, dass ein in die Handhabungseinrichtung (60) eingeführtes proximales Ende (51) einer zweiten Übertragungseinrichtung (50) an der Gleitfläche (103) gleitend den Riegel (100) entlang des weiteren Pfads (106) verschieben kann.

11. **Medizinisches Instrument** (10) mit einer Handhabungseinrichtung (60) nach einem der vorangehenden Ansprüche und einem Schaft (20), der einen Außenschaft (30), und in dem Außenschaft (30) eine erste Übertragungseinrichtung (40) und eine zweite Übertragungseinrichtung (50) umfasst.

12. Medizinisches Instrument (10) nach dem vorangehenden Anspruch, bei dem das proximale Ende (31) des Außenschafts (30) verhindert, dass die zweite Kopplungseinrichtung (84) ihre Entkopplungsposition (89) erreicht, wenn der Außenschaft mit dem Grundkörper in der vorgesehenen Weise verbunden ist.

13. Medizinisches Instrument (10) nach einem der Ansprüche 11 und 12, ferner mit:
einem ersten Werkzeug (14) und einem zweiten Werkzeug (16) am distalen Ende des Schafts (20),
wobei die **erste Betätigungseinrichtung** (70) der Handhabungseinrichtung (60) zum Öffnen und Schließen von Maulteilen des **ersten Werkzeugs** (14) ausgebildet ist und die **zweite Betätigungseinrichtung** (80) der Handhabungseinrichtung (60) zum Bewegen des **zweiten Werkzeugs** (16) ausgebildet ist.

14. Medizinisches Instrument (10) nach einem der Ansprüche 11 bis 13, bei dem
die zweite Übertragungseinrichtung einen Innenschaft (50) umfasst, wobei das **proximale Ende** (51) des Innenschafts (50) von einem metallischen Bauelement (56) gebildet wird,
wobei das metallische Bauelement (56) von einem mittleren Abschnitt (58) des Innenschafts (50) **elektrisch isoliert** ist.

15. Medizinisches Instrument (10) nach einem der Ansprüche 11 bis 14, bei dem
die zweite Übertragungseinrichtung einen Innenschaft (50) in dem Außenschaft (30) umfasst,
die erste Übertragungseinrichtung eine Übertragungsstange (40) in dem Innenschaft (50) umfasst,
wobei der Innenschaft (50) und die Übertragungsstange (40) so ausgebildet sind, dass ein elektrisch leitfähiges proximales Ende (51) des Innenschafts (50) in jeder Position zumindest entweder von dem Außenschafts (30) oder von der Übertragungsstange (40) **elektrisch isoliert** ist.

## Claims

1. Handling device (60) for a medical instrument (10), comprising
a main body (61) with a coupling (62) for releasable mechanical connection to a proximal end (31) of an outer shaft (30) provided for the medical instrument (10);
a first actuation device (70), which is movable relative to the main body (61);
a second actuation device (80), which is movable relative to the main body (61); a first coupling device (74) for coupling the first actuation device (70) to a first transfer device (40) for transferring at least either a force or a torque;
a second coupling device (84) for coupling the second actuation device (80) to a second transfer device (50) for transferring at least either a force or a torque;
wherein the first coupling device (74) and the second coupling device (84; 90) each have a decoupling position (79, 89), in which the associated actuation device (70, 80) is decoupled from the associated transfer device (40, 50),
wherein the decoupling positions (79, 89) of both coupling devices (74, 84) can then only be reached when the outer shaft (30) provided for the medical instrument (10) is not connected to the coupling (62) on the main body (61) in the manner provided for the use of the medical instrument.

2. Handling device (60) according to the preceding claim, wherein the second coupling device (84) is electrically insulated in any position at least either from the proximal end (31) of an outer shaft (30) provided for the medical instrument (10) and connected to the handling device (60) or from the proximal end (41) of a first transfer device (40) coupled to the first actuation device (70).

3. Handling device (60) according to one of the preceding claims, wherein the first actuation device (70) is arranged proximally of a stationary grip part (63) of the handling device (60), and the second actuation device (80) is arranged distally of the stationary grip part (63).

4. Handling device (60) according to one of the preceding claims, wherein the second coupling device (84) comprises a carriage (90), which is displaceable by means of the second actuation device (80) along a predetermined path (91).

5. Handling device (60) according to the preceding claim, wherein
the second coupling device (84) comprises a bolt (94) for holding the proximal end of the second transfer device (50) in an interlocked manner on the carriage (90),
the main body (61) is designed, as the second coupling device (84) approaches the decoupling position (89), to move the bolt (94) into an unlocking position (99), in which the bolt (94) no longer holds the proximal end (51) of the second transfer device (50) in an interlocked manner.

6. Handling device (60) according to the preceding claim, wherein
the carriage (90) is movable in a first direction (91),
the bolt (94) is movable relative to the carriage (90) in a second direction (92) perpendicular with respect to the first direction (91).

7. Handling device (60) according to one of Claims 1 to 4, wherein the second coupling device (84) is movable by means of the second actuation device (80) along a curved path (101).

8. Handling device (60) according to the preceding claim, wherein
an interlocked coupling of the second coupling device (84) to the second transfer device (50) is dependent on a spacing between the second coupling device (84) and a longitudinal axis (28) of the second transfer device (50),
the decoupling position (89) of the second coupling device (84) over the curved path (101) is distanced so far from the longitudinal axis (28) that there is no coupling between the second coupling device (84) and the second transfer device,
other positions (87, 88) of the second coupling device (84) are arranged so close to the longitudinal axis (28) that an interlocked coupling of the second coupling device (84) to the second transfer device (50) may be present.

9. Handling device (60) according to one of the preceding claims, wherein
the second coupling device (84) comprises a bolt (100),
wherein the bolt (100) is displaceable along the curved path (101) by actuation of the second actuation device (80),
wherein the bolt (100) is movable relative to the second actuation device (80) along a further path (106), which is substantially perpendicular with respect to the curved path (101).

10. Handling device (60) according to the preceding claim, wherein
the bolt (105) is movable out of a working position (108), relative to the second actuation device (80) along the further path (106) against a resilient force of a resilient element (105),
the bolt (100) has a sliding surface (103), which is inclined with respect to the curved path (101) and with respect to the further path (106) and is designed and arranged such that a proximal end (51), introduced into the handling device (60), of a second transfer device (50) can slide the bolt (100) along the further path (106) at the sliding surface (103).

11. Medical instrument (10) comprising a handling device (60) according to one of the preceding claims and a shaft (20), which comprises an outer shaft (30) and in the outer shaft (30) a first transfer device (40) and a second transfer device (50).

12. Medical instrument (10) according to the preceding claim, wherein the proximal end (31) of the outer shaft (30) prevents the second coupling device (84) from reaching its decoupling position (89) when the outer shaft is connected to the main body in the provided manner.

13. Medical instrument (10) according to one of Claims 11 and 12, furthermore comprising:
a first tool (14) and a second tool (16) at the distal end of the shaft (20),
wherein the first actuation device (70) of the handling device (60) is designed to open and close mouth parts of the first tool (14), and the second actuation device (80) of the handling device (60) is designed to move the second tool (16).

14. Medical instrument (10) according to one of Claims 11 to 13, wherein
the second transfer device comprises an inner shaft (50), wherein the proximal end (51) of the inner shaft (50) is formed by a metal component (56),
wherein the metal component (56) is electrically insulated from a central portion (58) of the inner shaft (50).

15. Medical instrument (10) according to one of Claims 11 to 14, wherein
the second transfer device comprises an inner shaft (50) in the outer shaft (30),
the first transfer device comprises a transfer rod (40) in the inner shaft (50),
wherein the inner shaft (50) and the transfer rod (40) are designed such that an electrically conductive proximal end (51) of the inner shaft (50) is electrically insulated in any position at least either from the outer shaft (30) or from the transfer rod (40).

## Revendications

1. Dispositif de manipulation (60) destiné à un instrument médical (10), ledit dispositif comprenant
un corps de base (61) pourvu d'un coupleur (62) destiné à établir une liaison mécanique amovible avec une extrémité proximale (31) d'une tige extérieure (30) prévue pour l'instrument médical (10) ;
un premier dispositif d'actionnement (70) qui est mobile par rapport au corps de base (61) ;
un deuxième dispositif d'actionnement (80) qui est mobile par rapport au corps de base (61) ;
un premier dispositif d'accouplement (74) destiné à accoupler le premier dispositif d'actionnement (70) à un premier dispositif de transmission (40) pour transmettre au moins une force ou un couple ;
un deuxième dispositif d'accouplement (84) destiné à accoupler le deuxième dispositif d'actionnement (80) à un deuxième dispositif de transmission (50) pour transmettre au moins une force ou un couple ;
le premier dispositif d'accouplement (74) et le deuxième dispositif d'accouplement (84 ; 90) comportant chacun une position de désaccouplement (79, 89) dans laquelle le dispositif d'actionnement (70, 80) associé est désaccouplé du dispositif de transmission (40, 50) associé,
les positions de désaccouplement (79, 89) des deux dispositifs d'accouplement (74, 84) ne pouvant être atteintes que si la tige extérieure (30) prévue pour l'instrument médical (10) n'est pas reliée au coupleur (62) au niveau du corps de base (61) de la manière prévue pour utiliser l'instrument médical.

2. Dispositif de manipulation (60) selon la revendication précédente, dans lequel le deuxième dispositif d'accouplement (84) est isolé électriquement, dans chaque position, au moins de l'extrémité proximale (31) d'une tige extérieure (30) prévue pour l'instrument médical (10) et reliée au dispositif de manipulation (60) ou de l'extrémité proximale (41) d'un premier dispositif de transmission (40) accouplé au premier dispositif d'actionnement (70).

3. Dispositif de manipulation (60) selon l'une des revendications précédentes, dans lequel le premier dispositif d'actionnement (70) est disposé du côté proximal d'une partie formant poignée fixe (63) du dispositif de manipulation (60) et le deuxième dispositif d'actionnement (80) est disposé du côté distal de la partie formant poignée fixe (63).

4. Dispositif de manipulation (60) selon l'une des revendications précédentes, dans lequel le deuxième dispositif d'accouplement (84) comprend un chariot (90) qui peut coulisser le long d'un chemin prédéterminé (91) au moyen du deuxième dispositif d'actionnement (80).

5. Dispositif de manipulation (60) selon la revendication précédente, dans lequel
le deuxième dispositif d'accouplement (84) comprend un verrou (94) destiné à maintenir l'extrémité proximale du deuxième dispositif de transmission (50) sur le chariot (90) par complémentarité de formes,
le corps de base (61) est conçu pour déplacer, lorsque le deuxième dispositif d'accouplement (84) approche de la position de désaccouplement (89), le verrou (94) jusque dans une position de déverrouillage (99) dans laquelle le verrou (94) ne maintient plus l'extrémité proximale (51) du deuxième dispositif de transmission (50) par complémentarité de formes.

6. Dispositif de manipulation (60) selon la revendication précédente, dans lequel
le chariot (90) est mobile dans une première direction (91),
le verrou (94) est mobile par rapport au chariot (90) dans une deuxième direction (92) perpendiculaire à la première direction (91).

7. Dispositif de manipulation (60) selon l'une des revendications 1 à 4, dans lequel le deuxième dispositif d'accouplement (84) est mobile le long d'un chemin incurvé (101) au moyen du deuxième dispositif d'actionnement (80).

8. Dispositif de manipulation (60) selon la revendication précédente, dans lequel
un accouplement par complémentarité de formes du deuxième dispositif d'accouplement (84) au deuxième dispositif de transmission (50) dépend d'une distance entre le deuxième dispositif d'accouplement (84) et un axe longitudinal (28) du deuxième dispositif de transmission (50),
la position de désaccouplement (89) du deuxième dispositif d'accouplement (84) sur le chemin incurvé (101) est si éloignée de l'axe longitudinal (28) qu'il n'y a pas d'accouplement entre le deuxième dispositif d'accouplement (84) et le deuxième dispositif de transmission,
d'autres positions (87, 88) du deuxième dispositif d'accouplement (84) sont si proches de l'axe longitudinal (28) qu'il peut y avoir un accouplement par complémentarité de formes du deuxième dispositif d'accouplement (84) au deuxième dispositif de transmission (50).

9. Dispositif de manipulation (60) selon l'une des revendications précédentes, dans lequel
le deuxième dispositif d'accouplement (84) comprend un verrou (100),
le verrou (100) pouvant coulisser le long du chemin incurvé (101) par actionnement du deuxième dispositif d'actionnement (80),
le verrou (100) étant mobile par rapport au deuxième dispositif d'actionnement (80) le long d'un autre chemin (106) qui est sensiblement perpendiculaire au chemin incurvé (101).

10. Dispositif de manipulation (60) selon la revendication précédente, dans lequel
le verrou (105) est mobile par rapport au deuxième dispositif d'actionnement (80) le long de l'autre chemin (106) en s'opposant à une force élastique d'un élément élastique (105) depuis une position de travail (108),
le verrou (100) comporte une surface de glissement (103) qui est inclinée par rapport au chemin incurvé (101) et par rapport à l'autre chemin (106) et est conçu et disposé de telle sorte qu'une extrémité proximale (51), insérée dans le dispositif de manipulation (60), d'un deuxième dispositif de transmission (50) peut faire coulisser le verrou (100) le long de l'autre chemin (106) en glissant sur la surface de glissement (103).

11. Instrument médical (10) comprenant un dispositif de manipulation (60) selon l'une des revendications précédentes et une tige (20) qui comprend une tige extérieure (30) et, dans la tige extérieure (30), un premier dispositif de transmission (40) et un deuxième dispositif de transmission (50).

12. Instrument médical (10) selon la revendication précédente, dans lequel l'extrémité proximale (31) de la tige extérieure (30) empêche le deuxième dispositif d'accouplement (84) d'atteindre sa position de désaccouplement (89) lorsque la tige extérieure est reliée au corps de base de la manière prévue.

13. Instrument médical (10) selon l'une des revendications 11 et 12, comprenant en outre :
un premier outil (14) et un deuxième outil (16) à l'extrémité distale de la tige (20),
le premier dispositif d'actionnement (70) du dispositif de manipulation (60) étant conçu pour ouvrir et fermer des parties de mâchoire du premier outil (14) et le deuxième dispositif d'actionnement (80) du dispositif de manipulation (60) étant conçu pour déplacer le deuxième outil (16).

14. Instrument médical (10) selon l'une des revendications 11 à 13, dans lequel
le deuxième dispositif de transmission comprend une tige intérieure (50), l'extrémité proximale (51) de la tige intérieure (50) étant formée par un composant métallique (56),
le composant métallique (56) étant isolé électriquement d'une portion centrale (58) de la tige intérieure (50).

15. Instrument médical (10) selon l'une des revendications 11 à 14, dans lequel
le deuxième dispositif de transmission comprend une tige intérieure (50) dans la tige extérieure (30),
le premier dispositif de transmission comprend une barre de transmission (40) dans la tige intérieure (50),
la tige intérieure (50) et la barre de transmission (40) étant conçues de telle sorte qu'une extrémité proximale (51) électriquement conductrice de la tige intérieure (50) soit isolée électriquement de la tige extérieure (30) ou de la barre de transmission (40) dans chaque position.
